# EUROPEAN PATENT APPLICATION

(11) **EP 2 557 163 A1**
(43) Date of publication of application: **13.02.2013**
(21) Application number: 11177451.9
(22) Date of filing: 12.08.2011
(51) Int. Cl.: C12N 15/74

(54) **Bacterially formed microcin S, a new antimicrobial peptide, effective against pathogenic microorganisms, e.g. enterohemorrhagic Escherichia coli (EHEC)**

(71) Applicant: SymbioGruppe GmbH & Co KG, 35745 Herborn (DE)
(72) Inventor: Gunzer, Florian, 01309 Dresden (DE); Zschüttig, Anke, 01159 Dresden (DE); Zimmermann, Kurt, 35745 Herborn (DE)
(74) Representative: Isarpatent

(57) **Abstract**

The present invention relates to a new isolated polypeptide nominated microcin S, isolated nucleic acid molecules encoding the microcin S polypeptide and primers and probes hybridizing to the nucleic acid molecules. The invention also relates to plasmids and cells comprising the nucleic acid molecules, an antibody binding to the polypeptide, compositions as well as methods for producing and using the polypeptides. The present invention further relates to medical uses for treating or preventing microbial infections, functional gastrointestinal disorders or treating a tumor. The invention further relates to a method for preserving food and a method for coating dressing material.

SEQUENCE LISTING

<110>  Symbio Gruppe GmbH & Co KG

<120>  BACTERIALLY FORMED MICROCIN S, A NEW ANTIMICROBIAL PEPTIDE,
       EFFECTIVE AGAINST PATHOGENIC MICROORGANISMS, E.G. EHEC

<130>  P31501-EP

<160>  26

<170>  PatentIn version 3.5

<210>  1
<211>  4679
<212>  DNA
<213>  Escherichia coli

<400>  1
ttaatttttg ttcagcccat catcaatggt gcgcttgtgc ctcagctcca taaagcagcc      60

gtctgccaga tacagaaccc ggtctgcaga ggctatggtt tccgggcgat gggctatcag     120

caggacagga agccccagct gacgtagtgt ctggcttatc tgaatttcac tttcaacatc     180

aagatggctg gtcgcttcat ccagtaataa aaatcccggt ttcttgtaca aagccctggc     240

cagcagaatg cgttgttttt gccctccgga aagccctcca ccggtttcac caatcagcgt     300

ctgatacccc atcggcatag acatgatatc gctgtcgata agtgccagtc tggcgcactg     360

gatcattcgt tcatgattgc ggtctccact gaaaaaaatg atgttatctg caatggagcc     420

cctgaaaaga tggtcatcct gcagtacggt accgattcgc tgacgtacct gaaaataatc     480

cgggtgggta tgcggtatgc caaatgccct gattgttccc tcatccggga tgtggatccc     540

caggataagc ttcaccagtg tcgatttccc gcatccggat tgaccggtaa tggccatcac     600

ctcgccagga tggagtgtca gcgacacccc gcggagtatc ggtttattcc ctcccttgtg     660

gctgaaggtt atatgctcaa gggataaagg aacacgggca ccgtcggcca catccccccg     720

gaatacggac gggaagacag aagtatcatt gtcttcctga gcgggttgtt gctgatagtc     780

ttctgtcggg gtcagcacaa tgtcagccag gcgttcgttg tagatatcaa gcatacgcca     840

ggaaaaatac ttatcggtca ggctgctgat actggatgaa aaacgcatct ggtaggacaa     900

ataagcaacc agcatgccga cggtgaaggt accgttcagt acttccccgg cacctttcca     960

caaaataatc gctgatacca cgctgccggt cagggtgtgt gcaatgtcat agcacatcag    1020

ctgacggctc tgtcgcagct gcgtattcct gcgaacaatg ttgaggttca gccaggcggc    1080

ctcacggtgc gctgtcacgc cgttaattct caggctcaga ataccgttga gggtttcaag    1140

aaaatgcccg gactccctgg cacctgcgtc ccaggcatct tctgccgact gtcgaaggga    1200

cggataccac aacgccctca gcacaccgta gataatggct gccagtacgg ctatcagcgt    1260

catttccgga ctgtacagca gcatcataca gagcgcagtt atcacgagca acacgtccag 1320

aataccttcc agtacctgcg tggtcagcgc ctcctggatg gtatccacgg cctcaaaacg 1380

ggcattgata cttcccttac tgcgcgcatc aaaccagccc agcgggagac gcaccagatg 1440

atggaaaacc cgggctgtcc actgcatgtt aaaattgacg gacagagtta tactggccca 1500

ctggcgtgcc agcgacagca gcatctgggt cattgacagc agcagcaggg caatgattat 1560

aacggtcagg agacttcgat cggcggcaac cagcacttca tcaattacca gctggttgag 1620

aagtggacta ctcagcgtca ggatttccag cgccagggca aaaatgatga tgcgcgacat 1680

cgcggccagc agcccgggtg tcctgcctgt cagttggcgc aggcgaattt tttttctttc 1740

atcccgggcg gtaaaatcac tggccggcgt caactccagc gcgacaccgg tgaaatgttt 1800

tcctgcatcc tgcagggaga gcgttatctt ccctttgtcc gggtcgtgga taaccagcct 1860

gctcccgcga actttataaa gcacgacgaa atggttcatg ttccagtgga gaatgcacgg 1920

gagagaaagg gatttcaggt cttccggctc cagccggacg gcccgggagg acaaccggat 1980

accggctgca cactctatca gcctctgcag ggtcatcccc tgtgtgctga tactgaaacg 2040

ttcgcgcagg gtgggtaaat ctgtctgcag accatgccag caggccatca tgaccagaca 2100

tgccagacca cattccgcag actctgtctg ccggatgagg gggagctgtt ttctcccggt 2160

ccatttaagg gtatccatta cagttttcct ttcaggctcc agagtggctc ggtcagccac 2220

tcccacaaat ggcgggtatc caggctgaca tccccctcaa gggtcatgcc ggggcgcagg 2280

ggttcctgtt ttccataagc cagaatgaag gtattctcag gttcgacgat gacgcggtag 2340

tgcccttcat tctctttcca ggtcacaggg gatacggaga gtaaatccga aggggtcagc 2400

gttgtacggc ttatctctcg gatagtcccg tactgaacgc caaacttctg ataggggaat 2460

gcggcgaacc gaagtgcaac acgctgcccc ggctgaataa aaccggcatt ctgactggtg 2520

gcatagagtt ctatctgcag acgagcattg tcgggaacca gagtcatgac cggttcagat 2580

gccctgacag actgtccctg tctgatcagc acggcagcaa cggttccgga taccggtgac 2640

gtcagcgtaa agttctcctg tccggccagt tcgtcctgct gttgcctgat cccctgcaac 2700

tgcctgtcca gctcagcttt acggctttcc ccctgaacga tgagatggtt aagatcttct 2760

tcagcagcct ccattgcagt gtgtagttgc agaagcccct gacgctgatc ctcaacgttt 2820

tgcagtgacg tagaaacgtc aatttgcttc tgctggtatt cgatatctga tacgtaatgc 2880

gttccagcca gttttttata gcgctccatg acggacacgg ccagcgtggc ctgatgcccg 2940

gcaagcgaca ggcgcagctc tgcactttta atctgtggtt tcagggaggc aattcgctgt 3000

agtatggcct gctgctgctg actgttgtca cgcaattcca gagtctgctg ggaagacagc 3060

atggcatact gagtcttcag ggacatgctc atggctgcca gtgtacctgt gccctgcccg 3120

ttgtaatgct cgccgctgat atggtaaaca ggctcaccgg cagctacgtg ctgaccttct 3180

gaaacagtct gtcgggtgac gtatccggca tattgcggtg tgattttcac cagtcctgat 3240

gaaggcatca ctatacctgt aagatgagcc ttgcgggtat aactaccata ataaacgaat 3300

aatgagacgc tcagaaatat aagtaatgta gcaacggcgc atactgacat gccaaatgac 3360

gcgggtaaga caatatctcc gtactctgta tcactgttat gttctaatgc ttcctccctg 3420

aatatactca tggttataaa atattttgaa tatttaataa tataacaaag gtattcatgc 3480

ctgaatgtat taacatagga taaaaaaggg atctgctttt aattctgacc cctaataaca 3540

gcatagatac aacaaaaagg actgattgat ccagaacatt ataatactga aagtgcatca 3600

gagagaaaaa caaagatgtt atcacacagg gaatgataat gttttccttg aaaattgaac 3660

gcagaaatcc aaaggcacag gctctgtata cgatctcctc atagtagggt acgaccagtg 3720

tcatcactaa aactgttatc cagtttatct gtgatggttc gtaatgatat aaatagtctc 3780

tgtagcagta aacagctaac tgtatcagaa taattataag gaatgacagg gctgaaaatt 3840

taactgtagc tgagtctgtt cttattttta ttctacactc aggaaattcc tttaggtaaa 3900

ataagtaaac aagagttgaa attagaatct ctgtaatgaa cacaatgaaa aatgcaatat 3960

ccagtccaac ataacgcagt gtaaaggttg gcgataatgt tacaatagtt gatataataa 4020

ccactgcaag aaaaataata gcgctgtatt tactatatct aaactggctc gaacgttcat 4080

ccattacaaa acatcacttt atgaaggtta ttatttttgc taaaactata tctgtaaatt 4140

tagcagtgga aaacaaggtg atcaatgtaa ttactatttt tgaaaattcc tcaggcaacg 4200

aaaaccatga caaatcaata atggaggaac cacttaatag cgcaaaaccc agtaataaac 4260

ttaatataaa agacaataat ttaataaaca ctgcagtatc ctccgttgat acttacttat 4320

cgactacagg taccaccgac gttacttcct gaacaattac tggaaccagc tctgtttccg 4380

cctccgttac catttcctcc accggagaga cactggccga taactgcccc accaatagtt 4440

cctctggtca ttcctacagg accaccttta atggcaccac caaccattcc actaaagaca 4500

gcattagcac atttaactgt gctcgggtca ctgtaaatgt gtgtcggtgc atttcgtccc 4560

agtgagttac gggctccggt gttacgacta cgacttccgc ctccttcata attactgttt 4620

gcgttgccac cactgactaa tgcaatttca tcaaaactca attctctgat atttgacat 4679

<210> 2
<211> 363
<212> DNA
<213> Escherichia coli

<400>  2
atgtcaaata tcagagaatt gagttttgat gaaattgcat tagtcagtgg tggcaacgca      60

aacagtaatt atgaaggagg cggaagtcgt agtcgtaaca ccggagcccg taactcactg     120

ggacgaaatg caccgacaca catttacagt gacccgagca cagttaaatg tgctaatgct     180

gtctttagtg gaatggttgg tggtgccatt aaaggtggtc ctgtaggaat gaccagagga     240

actattggtg gggcagttat cggccagtgt ctctccggtg gaggaaatgg taacggaggc     300

ggaaacagag ctggttccag taattgttca ggaagtaacg tcggtggtac ctgtagtcga     360

taa                                                                   363

<210>  3
<211>  651
<212>  DNA
<213>  Escherichia coli

<400>  3
atggatgaac gttcgagcca gtttagatat agtaaataca gcgctattat ttttcttgca      60

gtggttatta tatcaactat tgtaacatta tcgccaacct ttacactgcg ttatgttgga     120

ctggatattg catttttcat tgtgttcatt acagagattc taatttcaac tcttgtttac     180

ttattttacc taaaggaatt tcctgagtgt agaataaaaa taagaacaga ctcagctaca     240

gttaaatttt cagccctgtc attccttata attattctga tacagttagc tgtttactgc     300

tacagagact atttatatca ttacgaacca tcacagataa actggataac agttttagtg     360

atgacactgg tcgtacccta ctatgaggag atcgtataca gagcctgtgc ctttggattt     420

ctgcgttcaa ttttcaagga aaacattatc attccctgtg tgataacatc tttgtttttc     480

tctctgatgc actttcagta ttataatgtt ctggatcaat cagtcctttt tgttgtatct     540

atgctgttat taggggtcag aattaaaagc agatcccttt tttatcctat gttaatacat     600

tcaggcatga atacctttgt tatattatta aatattcaaa atattttata a             651

<210>  4
<211>  1254
<212>  DNA
<213>  Escherichia coli

<400>  4
atgagtatat tcagggagga agcattagaa cataacagtg atacagagta cggagatatt      60

gtcttacccg cgtcatttgg catgtcagta tgcgccgttg ctacattact tatatttctg     120

agcgtctcat tattcgttta ttatggtagt tatacccgca aggctcatct tacaggtata     180

gtgatgcctt catcaggact ggtgaaaatc acaccgcaat atgccggata cgtcacccga     240

cagactgttt cagaaggtca gcacgtagct gccggtgagc ctgtttacca tatcagcggc 300

gagcattaca acgggcaggg cacaggtaca ctggcagcca tgagcatgtc cctgaagact 360

cagtatgcca tgctgtcttc ccagcagact ctggaattgc gtgacaacag tcagcagcag 420

caggccatac tacagcgaat tgcctccctg aaaccacaga ttaaaagtgc agagctgcgc 480

ctgtcgcttg ccgggcatca ggccacgctg gccgtgtccg tcatggagcg ctataaaaaa 540

ctggctggaa cgcattacgt atcagatatc gaataccagc agaagcaaat tgacgtttct 600

acgtcactgc aaaacgttga ggatcagcgt caggggcttc tgcaactaca cactgcaatg 660

gaggctgctg aagaagatct taaccatctc atcgttcagg gggaaagccg taaagctgag 720

ctggacaggc agttgcaggg gatcaggcaa cagcaggacg aactggccgg acaggagaac 780

tttacgctga cgtcaccggt atccggaacc gttgctgccg tgctgatcag acagggacag 840

tctgtcaggg catctgaacc ggtcatgact ctggttcccg acaatgctcg tctgcagata 900

gaactctatg ccaccagtca gaatgccggt tttattcagc cggggcagcg tgttgcactt 960

cggttcgccg cattccccta tcagaagttt ggcgttcagt acgggactat ccgagagata 1020

agccgtacaa cgctgacccc ttcggattta ctctccgtat cccctgtgac ctggaaagag 1080

aatgaagggc actaccgcgt catcgtcgaa cctgagaata ccttcattct ggcttatgga 1140

aaacaggaac ccctgcgccc cggcatgacc cttgaggggg atgtcagcct ggatacccgc 1200

catttgtggg agtggctgac cgagccactc tggagcctga aaggaaaact gtaa 1254

<210> 5
<211> 2178
<212> DNA
<213> Escherichia coli

<400> 5
atggataccc ttaaatggac cgggagaaaa cagctccccc tcatccggca gacagagtct 60

gcggaatgtg gtctggcatg tctggtcatg atggcctgct ggcatggtct gcagacagat 120

ttacccaccc tgcgcgaacg tttcagtatc agcacacagg ggatgaccct gcagaggctg 180

atagagtgtg cagccggtat ccggttgtcc tcccgggccg tccggctgga gccggaagac 240

ctgaaatccc tttctctccc gtgcattctc cactggaaca tgaaccattt cgtcgtgctt 300

tataaagttc gcgggagcag gctggttatc cacgacccgg acaaagggaa gataacgctc 360

tccctgcagg atgcaggaaa acatttcacc ggtgtcgcgc tggagttgac gccggccagt 420

gattttaccg cccgggatga aagaaaaaaa attcgcctgc gccaactgac aggcaggaca 480

cccgggctgc tggccgcgat gtcgcgcatc atcatttttg ccctggcgct ggaaatcctg 540

acgctgagta gtccacttct caaccagctg gtaattgatg aagtgctggt tgccgccgat 600

cgaagtctcc tgaccgttat aatcattgcc ctgctgctgc tgtcaatgac ccagatgctg 660

ctgtcgctgg cacgccagtg ggccagtata actctgtccg tcaattttaa catgcagtgg 720

acagcccggg ttttccatca tctggtgcgt ctcccgctgg gctggtttga tgcgcgcagt 780

aagggaagta tcaatgcccg ttttgaggcc gtggatacca tccaggaggc gctgaccacg 840

caggtactgg aaggtattct ggacgtgttg ctcgtgataa ctgcgctctg tatgatgctg 900

ctgtacagtc cggaaatgac gctgatagcc gtactggcag ccattatcta cggtgtgctg 960

agggcgttgt ggtatccgtc ccttcgacag tcggcagaag atgcctggga cgcaggtgcc 1020

agggagtccg ggcattttct tgaaaccctc aacggtattc tgagcctgag aattaacggc 1080

gtgacagcgc accgtgaggc cgcctggctg aacctcaaca ttgttcgcag gaatacgcag 1140

ctgcgacaga gccgtcagct gatgtgctat gacattgcac acaccctgac cggcagcgtg 1200

gtatcagcga ttattttgtg gaaaggtgcc ggggaagtac tgaacggtac cttcaccgtc 1260

ggcatgctgg ttgcttattt gtcctaccag atgcgttttt catccagtat cagcagcctg 1320

accgataagt atttttcctg gcgtatgctt gatatctaca acgaacgcct ggctgacatt 1380

gtgctgaccc cgacagaaga ctatcagcaa caacccgctc aggaagacaa tgatacttct 1440

gtcttcccgt ccgtattccg gggggatgtg gccgacggtg cccgtgttcc tttatccctt 1500

gagcatataa ccttcagcca caagggaggg aataaaccga tactccgcgg ggtgtcgctg 1560

acactccatc ctggcgaggt gatggccatt accggtcaat ccggatgcgg gaaatcgaca 1620

ctggtgaagc ttatcctggg gatccacatc ccggatgagg gaacaatcag ggcatttggc 1680

ataccgcata cccacccgga ttattttcag gtacgtcagc gaatcggtac cgtactgcag 1740

gatgaccatc ttttcagggg ctccattgca gataacatca tttttttcag tggagaccgc 1800

aatcatgaac gaatgatcca gtgcgccaga ctggcactta tcgacagcga tatcatgtct 1860

atgccgatgg ggtatcagac gctgattggt gaaaccggtg gagggctttc cggagggcaa 1920

aaacaacgca ttctgctggc cagggctttg tacaagaaac cgggattttt attactggat 1980

gaagcgacca gccatcttga tgttgaaagt gaaattcaga taagccagac actacgtcag 2040

ctggggcttc ctgtcctgct gatagcccat cgcccggaaa ccatagcctc tgcagaccgg 2100

gttctgtatc tggcagacgg ctgctttatg gagctgaggc acaagcgcac cattgatgat 2160

gggctgaaca aaaattaa 2178

<210> 6
<211> 120
<212> PRT
<213> Escherichia coli

<400>  6

Met Ser Asn Ile Arg Glu Leu Ser Phe Asp Glu Ile Ala Leu Val Ser
1               5                   10                  15

Gly Gly Asn Ala Asn Ser Asn Tyr Glu Gly Gly Gly Ser Arg Ser Arg
            20                  25                  30

Asn Thr Gly Ala Arg Asn Ser Leu Gly Arg Asn Ala Pro Thr His Ile
        35                  40                  45

Tyr Ser Asp Pro Ser Thr Val Lys Cys Ala Asn Ala Val Phe Ser Gly
    50                  55                  60

Met Val Gly Gly Ala Ile Lys Gly Gly Pro Val Gly Met Thr Arg Gly
65                  70                  75                  80

Thr Ile Gly Gly Ala Val Ile Gly Gln Cys Leu Ser Gly Gly Gly Asn
                85                  90                  95

Gly Asn Gly Gly Gly Asn Arg Ala Gly Ser Ser Asn Cys Ser Gly Ser
            100                 105                 110

Asn Val Gly Gly Thr Cys Ser Arg
        115                 120

<210>  7
<211>  102
<212>  PRT
<213>  Escherichia coli

<400>  7

Asn Ala Asn Ser Asn Tyr Glu Gly Gly Gly Ser Arg Ser Arg Asn Thr
1               5                   10                  15

Gly Ala Arg Asn Ser Leu Gly Arg Asn Ala Pro Thr His Ile Tyr Ser
            20                  25                  30

Asp Pro Ser Thr Val Lys Cys Ala Asn Ala Val Phe Ser Gly Met Val
        35                  40                  45

Gly Gly Ala Ile Lys Gly Gly Pro Val Gly Met Thr Arg Gly Thr Ile
    50                  55                  60

Gly Gly Ala Val Ile Gly Gln Cys Leu Ser Gly Gly Gly Asn Gly Asn
65                  70                  75                  80

Gly Gly Gly Asn Arg Ala Gly Ser Ser Asn Cys Ser Gly Ser Asn Val
                85                  90                  95

Gly Gly Thr Cys Ser Arg
            100

<210>  8
<211>  19
<212>  DNA
<213>  Artificial Sequence

<220>
<223>  pSYM1 sequencing primer

<400>  8
cagctggata tcctgcgcg                                                    19

<210>  9
<211>  19
<212>  DNA
<213>  Artificial Sequence

<220>
<223>  pSYM1 sequencing primer

<400>  9
ggttgcccgg catccaacg                                                    19

<210>  10
<211>  31
<212>  DNA
<213>  Artificial Sequence

<220>
<223>  pAZ6/pAZ8 cloning primer

<400>  10
tcaattgtgt cgactcaatt actcttgtga g                                      31

<210>  11
<211>  34
<212>  DNA
<213>  Artificial Sequence

<220>
<223>  pAZ6 cloning primer

<400>  11
catgtaatag tgctagcatg ttaaaattta taag                                   34

<210>  12
<211>  35

<212>  DNA
<213>  Artificial Sequence

<220>
<223>  pAZ8, pAZ12 cloning primer

<400>  12
caaaaataat agctagcaag tgatgttttg taatg                                35


<210>  13
<211>  28
<212>  DNA
<213>  Artificial Sequence

<220>
<223>  pAZ9 cloning primer

<400>  13
ctcgaattca tccattacaa aacatcac                                        28


<210>  14
<211>  30
<212>  DNA
<213>  Artificial Sequence

<220>
<223>  pAZ9 cloning primer

<400>  14
ctggctgcag taattgttca ggaagtaacg                                      30


<210>  15
<211>  27
<212>  DNA
<213>  Artificial Sequence

<220>
<223>  pAZ10 cloning primer

<400>  15
taggaattca gaggaactat tggtggg                                         27


<210>  16
<211>  37
<212>  DNA
<213>  Artificial Sequence

<220>
<223>  pAZ10 cloning primer

<400>  16
ctccgctgca gacttactta tcgactacag gtaccac                              37


<210>  17
<211>  37

<212>  DNA
<213>  Artificial Sequence

<220>
<223>  pAZ11 cloning primer

<400>  17
gttagaattc ataagaggga tttttatgtc aaatatc                                37


<210>  18
<211>  35
<212>  DNA
<213>  Artificial Sequence

<220>
<223>  pAZ11 cloning primer

<400>  18
gttgatactg cagcttatcg actacaggta ccacc                                  35


<210>  19
<211>  31
<212>  DNA
<213>  Artificial Sequence

<220>
<223>  pAZ12 cloning primer

<400>  19
cccaagctta gttaaatgtg ctaatgctgt c                                      31


<210>  20
<211>  18
<212>  DNA
<213>  Artificial Sequence

<220>
<223>  pAZ12 cloning primer

<400>  20
ggcatcggtc gacgcaac                                                     18


<210>  21
<211>  32
<212>  DNA
<213>  Artificial Sequence

<220>
<223>  pAZ14 cloning primer

<400>  21
cattgctagc catcacagat aaactggata ac                                     32


<210>  22
<211>  33

<212>  DNA
<213>  Artificial Sequence

<220>
<223>  pAZ13, pAZ14 cloning primer

<400>  22
ccctgagtcg actcatggtt ataaaatatt ttg                                    33


<210>  23
<211>  22
<212>  DNA
<213>  Artificial Sequence

<220>
<223>  inhibition control

<400>  23
atggctatcg acgaaaacaa ac                                                22


<210>  24
<211>  24
<212>  DNA
<213>  Artificial Sequence

<220>
<223>  inhibition control

<400>  24
ttaaaaatct tcgttagttt ctgc                                              24


<210>  25
<211>  23
<212>  DNA
<213>  Artificial Sequence

<220>
<223>  mcsS screening primer

<400>  25
atgtcaaata tcagagaatt gag                                               23


<210>  26
<211>  21
<212>  DNA
<213>  Artificial Sequence

<220>
<223>  mcsS screening primer

<400>  26
ttatcgacta caggtaccac c                                                 21

## Description

### Field of the invention

The present invention relates to an isolated polypeptide, isolated nucleic acid molecules encoding a microcin S polypeptide and primers and probes hybridizing to the nucleic acid molecules. The invention also relates to plasmids and cells comprising the nucleic acid molecules, an antibody binding to the polypeptide, compositions as well as methods for producing and using the polypeptides. The present invention further relates to medical uses for treating or preventing microbial infections, functional gastrointestinal disorders or treating a tumor. The invention further relates to a method for preserving food and a method for coating dressing material.

### Background of the invention

Microcins are ribosomally synthesized antimicrobial peptides with a low molecular mass. Produced by enterobacteria, mostly *Escherichia coli,* microcin synthesis is sharply activated under stress conditions, such as limitation of nutrients. Microcins exert potent antimicrobial/antibacterial activity against closely related species, which offers a highly competitive advantage in the intestinal microflora (Baquero, F., Bouanchaud, D., Martinez-Perez, M. C. & Fernandez, C. (1978) J Bacteriol 135, 342-347). Microcin-producers are resistant to the microcin they produce, which is mediated by at least one resistance-conferring gene located within one gene cluster. Most of the 14 known microcins are plasmid-encoded, but also chromosomally encoded antimicrobial/antibacterial peptides have been described. The probiotic strain *E. coli* Nissle 1917 (EcN) produces microcins M and H47 (Patzer, S. I., Baquero, M. R., Bravo, D., Moreno, F. & Hantke, K. (2003) Microbiology 149, 2557-2570.). Probiotics are defined as living microorganisms, which upon ingestion in certain numbers exert health benefits beyond inherent basic nutrition. The mechanisms that enable a strain to be probiotic are poorly understood. Nevertheless, the antimicrobial activity of microcins may positively influence the intestinal balance. Assuming the absence of pathogenic factors as well as well-proven clinical safety, a microcin-producing strain can clearly fulfill the definition of a probiotic. In contrast to enterobacterial microcins, food-borne lactic acid bacteria produce lanthionine-containing peptide antibiotics. The so-called lantibiotics of gram-positive bacteria are already used for food preservation (Kuipers, A., Rink, R. & Moll, G. N. (2011) Prokaryotic Antimicrobial Peptides: From Genes to Applications (Springer Science+Business Media, Berlin). The use as an antitumoral agent (Hetz, C., Bono, M. R., Barros, L. F. & Lagos, R. (2002) Proc Natl Acad Sci U S A 99, 2696-2701) or as an alternative to classical antibiotics in infectious diseases (Dicks, L. M. T., Heunis, T. D. J., van Staden, D. A., Brand, A., Sutyak Noll, K. & Chikindas, M. L. (2011) Prokaryotic Antimicrobial Peptides: From Genes to Applications (Springer Science+Business Media, Berlin), Gillor, O., Kirkup, B. C. & Riley, M. A. (2004) Adv. Appl Microbiol 54, 129-146) are two further discussed applications of bacteriocins.

Since decades and at present, the treatment of bacterial infections is mainly based on classical antibiotics. However, the emergence of pathogens resistant to these classical antibiotics constitutes a massive problem. Consequently, the remaining treatment options are even more restricted. This negative effect is also seen in other economically important industries as e.g. food industry.

### Summary of the invention

### Technical problem

The present invention was made in view of the prior art described above and in view of the increasing demand of new antibacterial and prophylactic agents in human and veterinary medicine. Therapy of enhanced-spectrum beta-lactamase (ESBL) strains is increasingly challenging. The object of the present invention is the provision of a new antimicrobial microcin polypeptide that can be used as an alternative to conventional antibiotics and thus to the control of pathogenic microorganisms that are ESBL-producers. In this regard, it is an object of the present invention to provide for a new antimicrobial microcin polypeptide that can be used in the treatment of pathogenic Gram negative Bacteria including ESBL-producing *E. coli,* and furthermore also including the highly virulent enterohemorrhagic *E. coli* (EHEC) strain that was the causative pathogen of a very recent large outbreak in Germany (Frank, C., et al. (2011) N Engl J Med 10.1056/NEJMoa1106483; Mellmann, A., et al. (2011) PLoS ONE 6(7): e22751. doi:10.1371). EHEC cells release higher amounts of Shiga toxins by treatment with certain antibiotics. Therefore, antibiotic-treatment of patients is discussed to be contraindicated. The new microcin polypeptide should be effective in the treatment and the prevention of EHEC and enteropathogenic *E. coli* (EPEC) infections. It is a further object of the invention to provide for a new antimicrobial microcin polypeptide that can be used in the treatment of functional gastrointestinal disorders, in particular irritable bowel syndrome in adults and children. It is a further object of the invention to provide for compositions that comprise the new microcin polypeptide of cells producing the new microcin polypeptide for the said medical uses. Object of the present invention is also to provide a novel microcin polypeptide that can be used in the treatment of cancer, that can be used in the preservation of food and can be used in the coating of dressing material.

### Disclosure of the invention

To solve the problem, the present invention features in one aspect an isolated polypeptide, wherein the polypeptide:
a) comprises an amino acid sequence which is at least 50% or more identical to the amino acid sequence of SEQ ID NO: 6 or of SEQ ID NO: 7;
b) is encoded by a nucleic acid molecule comprising a nucleotide sequence which is at least 50% or more identical to a nucleotide sequence comprising the nucleotide sequence of any one of SEQ ID NOs: 1, 2, 3, 4 or 5 or a complement thereof;
c) is encoded by a nucleic acid molecule which hybridizes to a nucleotide sequence complementary to a nucleotide sequence comprising the sequence of nucleotides of any one of SEQ ID NOs: 1, 2, 3, 4 or 5 or a complement thereof under stringent conditions; or
d) comprises a naturally occurring allelic variant of a polypeptide comprising the amino acid sequence of SEQ ID NO: 6 or of SEQ ID NO: 7.

In a further aspect of the invention, an isolated nucleic acid molecule encoding a microcin S polypeptide is made available, wherein said nucleic acid molecule:
a) comprises a nucleotide sequence that is at least 50% or more identical to any one of SEQ ID NOs: 1, 2, 3, 4 or 5 or a complement thereof,
b) comprises a nucleic acid molecule which hybridizes to a nucleotide sequence complementary to a nucleotide sequence comprising the nucleotide sequence of any one of SEQ ID NOs: 1, 2, 3, 4 or 5 or a complement thereof under stringent conditions;
c) comprises a nucleotide sequence that encodes a polypeptide comprising an amino acid sequence which is at least 50% or more identical to the amino acid sequence of SEQ ID NO: 6 or of SEQ ID NO: 7; or
d) comprises a nucleic acid molecule which encodes a naturally occurring allelic variant of a polypeptide comprising the amino acid sequence of SEQ ID NO: 6 or of SEQ ID NO: 7.

In various aspects, the invention relates to a plasmid comprising the nucleic acid molecule according to the invention and to a cell comprising the nucleic acid molecule and/or the polypeptide and/or the plasmid according to the invention.

The invention is also directed to an antibody which selectively binds to a polypeptide according to the invention.

A further aspect of the invention relates to a composition comprising
a) a polypeptide according to the invention; and/or
b) a cell according to the invention.

Also disclosed are the polypeptide, the cell and the composition of the invention for use in therapy or prevention.

In one aspect, the polypeptide, the cell and the composition of the invention are for use in treating or preventing microbial infections, preferably the microbial infection comprises infections with enteropathogenic and/or enterohemorrhagic *E. coli* (EPEC, EHEC) or preferably the microbial infection comprises hemolytic-uremic syndrome (HUS), preferably enteropathic hemolytic-uremic syndrome.

In another aspect the polypeptide, the cell and the composition of the invention are for use in treating gastrointestinal disorders, preferably functional gastrointestinal disorders.

In a further aspect, the polypeptide, the cell and the composition of the invention are for use in treating a tumor.

The invention features a method for producing the polypeptide of the invention, the method comprising:
using a nucleic acid molecule, a nucleic acid primer or probe, a plasmid or a cell according to the invention, or.
synthesizing the polypeptide of the invention by means of solution phase or solid phase peptide synthesis techniques.

The isolated polypeptides according to the invention can be produced by said method for producing the polypeptide.

In an additional aspect, an *in vitro* method for identifying bacteria which are sensitive to microcin S is provided, wherein the method comprises using a polypeptide; a nucleic acid molecule, a nucleic acid primer or probe, a cell, an antibody or a composition according to the invention.

The invention also relates to a method for preserving food, the method comprising:
adding to food at least one kind of a natural antimicrobial agent, wherein the agent is
   a) the polypeptide according to the invention;
   b) the cell according to the invention; and/or
   c) the composition according to the invention.

In a further aspect, the invention relates to a method for coating dressing material, wherein dressing material is coated with:
a) the polypeptide according to the invention; and/or
b) the composition according to the invention.

### Advantageous effects of invention

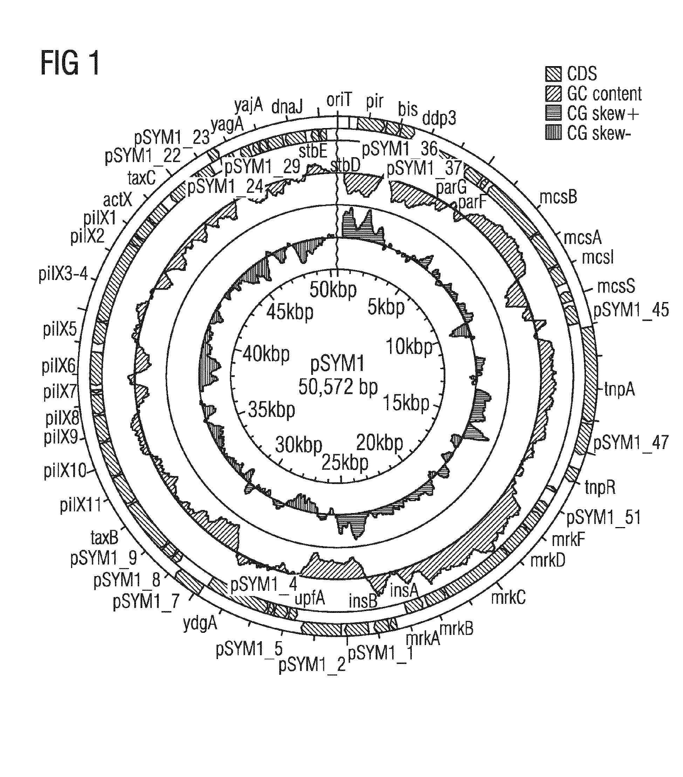

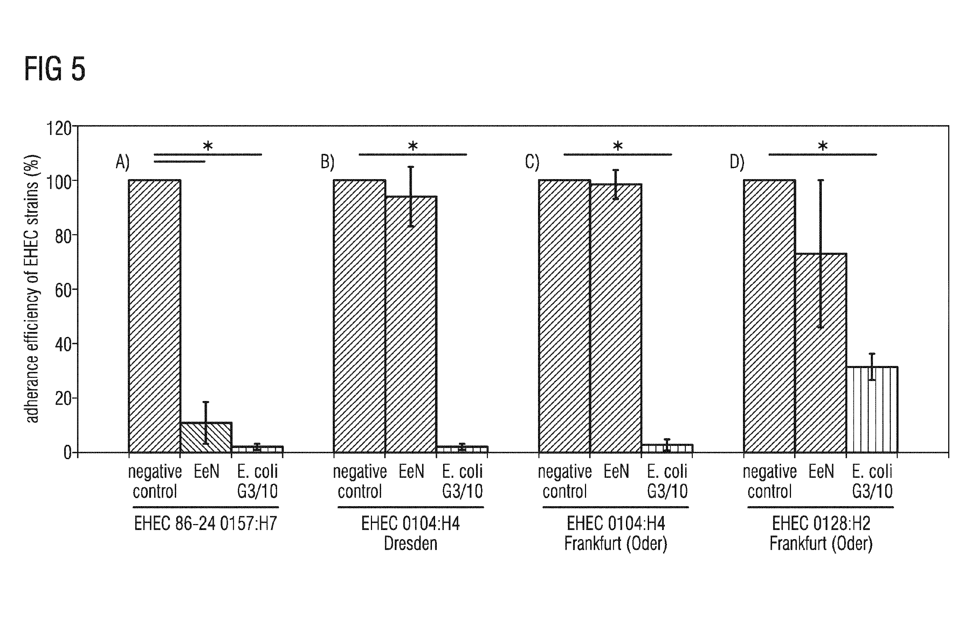

That is, the present inventors have identified the polypeptide microcin S which is a new, not previously described microcin, whose nucleotide and amino acid sequence is unique. The genes which are responsible for the effects of microcin S have been identified and their effects have been functionally characterized. The inventors have surprisingly found that, in contrast to most of the classical beta-lactam-antibiotics, microcin S shows an inhibitory effect of EHEC including the ESBL-producing O104:H4 outbreak strain as well as an O128:H2 and an O157:H7 EHEC isolate (Fig. 5). Thus, it has been further found that microcin S is effective in the treatment of enterohemorrhagic *E. coli* (EHEC) and enteropathogenic *E. coli* (EPEC) infection. Therefore, microcin S might be used as an alternative to conventional antibiotics and thus to the control of pathogenic microorganisms. Furthermore, a use for food preservation is possible, and the present invention was thereby completed. It has been further found that the microcin S (MccS) gene cluster (SEQ ID NO: 1) of *E. coli* G3/10, encoded by plasmid pSYM1 (Fig. 1), consists of the four clustered genes *mcsS* (SEQ ID NO: 2), *mcsI* (SEQ ID NO: 3), *mcsA* (SEQ ID NO: 4) and *mcsB* (SEQ ID NO: 5). Since *E. coli* G3/10 is a well-proven probiotic used for example for the treatment of gastrointestinal disorders, microcin S does not necessarily have to be purified from the strain. MccS should advantageously be effective *in vivo.*

Advantages are that the microcin S is nontoxic, may not induce allergies, and is difficult for pathogenic microbials to develop resistance against.

Other features and advantages of the invention will be apparent from the following detailed description, and from the claims.

### Brief description of the drawings

Fig. 1 is a vector map of megaplasmid pSYM1 encoding the MccS operon.
Fig. 2 is a schematic drawing of the amino acid sequence of gene *mcsS* encoding the microcin S precursor (upper panel). A probable leader peptide is underlined. Asterisks indicate Cys possibly involved in the formation of a disulfide bond typical for class II microcins. The microcin S gene cluster MccS (lower panel) on megaplasmid pSYM1 consists of the four clustered genes *mcsS, mcsI, mcsA* and *mcsB.*
Fig. 3 depicts adherence efficiency of EPEC E2348/69 into human intestinal epithelial cells after pre-incubation with *E. coli* strains MDS42 and G4/9 (wild-type) and appropriate plasmid-complementation mutants. Meaning of the used asterisk: * p ≤ 0.01.
Fig. 4 depicts the adherence efficiency of EPEC E2348/69 wild-type and plasmid-complemented mutants to human intestinal epithelial cells after pre-incubation with different microcin expressing and non-expressing strains. Meaning of the used asterisks: * p ≤ 0.01, ** p ≤ 0.05. pAZ8 [*mcsA, mcsB, mcsI*], pAZ13 [*mcsI*], pAZ14 [*mcsI,* truncated].
Fig. 5 shows the adherence efficiency of EHEC strains 86-24 serotype O157:H7 (A), EHEC O104:H4 isolated in Dresden (B) and EHECs O104:H4 (C) and O128:H2 (D) isolated in Frankfurt (Oder) into human intestinal epithelial cells after pre-incubation with EcN or *E. coli* G3/10. Meaning of the used asterisk: * p ≤ 0.01 compared to negative control.
Fig. 6 illustrates results of a *mcsS* screening using multiplex PCR and the gene *recA* as inhibition control.

### Brief description of the sequence listing

SEQ ID NO: 1 is the nucleotide sequence of the microcin S operon of *E. coli* G3/10.
SEQ ID NO: 2 is the nucleotide sequence of the gene *mcsS* of *E. coli* G3/10.
SEQ ID NO: 3 is the nucleotide sequence of the gene *mcsI* of *E. coli* G3/10.
SEQ ID NO: 4 is the nucleotide sequence of the gene *mcsA* of *E. coli* G3/10.
SEQ ID NO: 5 is the nucleotide sequence of the gene *mcsB* gene of *E. coli* G3/10.
SEQ ID NO: 6 is the amino acid sequence of the microcin S precursor polypeptide of *E. coli* G3/10 including a leader sequence.
SEQ ID NO: 7 is the amino acid sequence of the microcin S polypeptide of *E. coli* G3/10 without the leader peptide.
SEQ ID NOs: 8 - 26 are oligonucleotide primers used to amplify genes contained in the microcin S operon (SEQ ID NO: 1) or probes to screen for the *mcsS* gene (SEQ ID NO: 2) encoding the microcin S polypeptide.

### Detailed description of the invention

### Definitions

Unless otherwise defined, all technical and scientific terms used herein have the same meaning as commonly understood by the skilled person to which this invention belongs. In case of conflict, the present specification, including definitions, will control.

The articles "*a*", "*an*" or "*the*" relate to "*one*" or "*several*" whenever used herein, i.e. it means "*one*", "*at least one*" or "*one or more*". For example, the term "*a cell*" may refer to a single cell as well as to a plurality of cells.

The term "*comprise*" includes also embodiments wherein the term "*comprises*" means "*consists of*"*.*

As used herein amino acid or nucleic acid molecule "*identity*" is equivalent to amino acid or nucleic acid molecule "*homology*".

The phrase "*stringent conditions*" refers to conditions under which a probe nucleic acid molecule will hybridize to its target nucleic acid molecule sequence, typically in a complex mixture of nucleic acid molecules, but to no other sequences. Stringent conditions are sequence-dependent and will be different in different circumstances. Longer sequences hybridize specifically at higher temperatures. Nucleic acid hybridization parameters may be found in references which compile such methods, e.g., Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. For high stringency hybridization, a positive signal is at least two times background, preferably 10 times background hybridization. Exemplary high stringency or stringent hybridization conditions include: 50% formamide, 5x SSC and 1% SDS incubated at 42° C or 5x SSC and 1% SDS incubated at 65° C, with a wash in 0.2x SSC and 0.1% SDS at 65° C.

As used herein, the term "*microcin-like activity*" of a polypeptide means that the polypeptide exerts potent antimicrobial/antibacterial activity against closely related species. It does further mean that microcin-producers are resistant to the microcin they produce, which is mediated by at least one resistance-conferring gene located within one gene cluster.

As used herein, the term "*plasmid*" refers to a nucleic acid molecule capable of replication in a cell and to which another nucleic acid molecule can be operatively linked so as to bring about replication of the attached segment. Plasmids capable of directing the expression of a structural gene coding for a subject polypeptide are referred to herein as "expression plasmids."

As used herein, the phase "*operatively linked*" means that the subject nucleic acid molecule is attached to the plasmid so that expression of the structural gene formed by the nucleic acid molecule is under the control of the plasmid.

The term "*regulatory sequence*" is intended to include promoters, enhancers and other expression control elements (e.g., polyadenylation signals).

Herein, the term "*conjugative plasmid*" refers to a plasmid that can move from one cell to another during the process of conjugation.

As used herein, the terms "*transformation*" and "*transfection*" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

The term "*probiotic*" as used herein refers to living microorganisms, which upon ingestion in certain numbers, exert health benefits beyond inherent basic nutrition.

As used herein a "*pharmaceutical composition*" refers to a preparation of one or more of the active ingredients described herein, i.e. the polypeptide according to the invention (or a pharmaceutically acceptable salt thereof) or the cell according to the invention with other chemical components such as physiologically and pharmaceutically acceptable carriers and excipients. The purpose of a pharmaceutical composition is to facilitate administration of a compound to an organism.

Hereinafter, the phrases "*physiologically acceptable carrier*" and "*pharmaceutically acceptable carrier*" which may be interchangeably used refer to a carrier or a diluent that does not cause significant irritation to an organism and does not abrogate the biological activity and properties of the administered compound. One of the ingredients included in the pharmaceutically acceptable carrier can be for example polyethylene glycol (PEG), a biocompatible polymer with a wide range of solubility in both organic and aqueous media (Mutter *et al.* (1979).

Herein the term "*excipient*" refers to an inert substance added to a pharmaceutical composition to further facilitate administration of an active ingredient. Examples, without limitation, of excipients include calcium carbonate, calcium phosphate, various sugars and types of starch, cellulose derivatives, gelatine, vegetable oils and polyethylene glycols.

As used herein the term "*treating*" refers to preventing, curing, reversing, attenuating, alleviating, minimizing, suppressing or halting the deleterious effects of a disease process.

As used herein the term "*subject*" refers to a subject who may benefit from the present invention such as an animal or mammal (e.g., canine, feline, ovine, porcine, equine, bovine, human), preferably a human subject.

Herein, the term "*functional gastrointestinal disorder*" encompasses a number of separate idiopathic disorders which affect different part of the gastrointestinal tract.

### Polypeptides

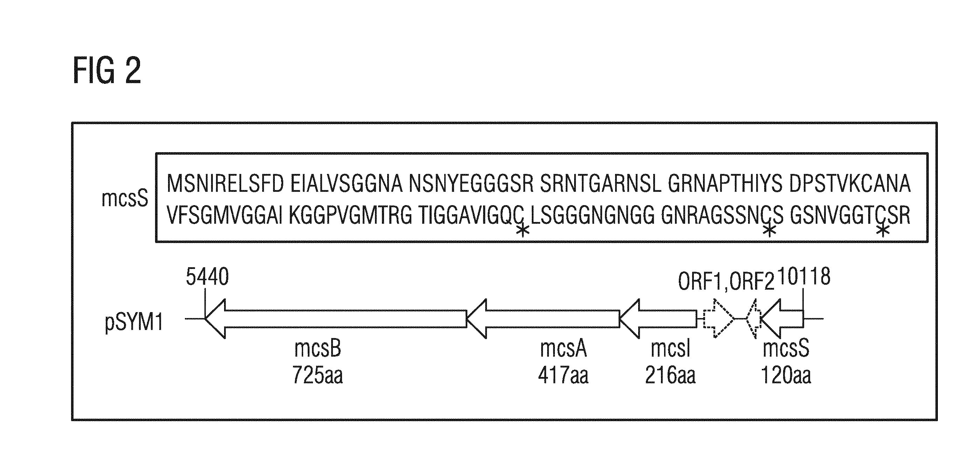

The microcin S polypeptide of the present invention is characterized by its amino acid residue sequence and novel functional properties. Polypeptides can be isolated using methods known in the art. That is, a naturally occurring or recombinantly produced microcin S polypeptide can be isolated from cells or tissue sources by an appropriate purification scheme using standard protein purification techniques. However, microcin S polypeptides may be naturally occurring in a cell or may be produced by recombinant DNA techniques or alternative to recombinant expression, a microcin S polypeptide can be synthesized chemically using standard peptide synthesis techniques. Herein, the microcin S polypeptide is classified as an *E. coli* microcin which has been phenotypically characterized and nominated as microcin S. Fig. 2 shows an amino acid sequence of microcin S precursor in the upper panel. A double glycine leader peptide is underlined. Asterisks indicate cysteins possibly involved in the formation of a disulfide bond typical for class II microcins. The amino acid sequence depicts a glycine-rich peptide with a double-glycine cleavage site. Polypeptides having the amino acid sequence of SEQ ID NO: 6 or of SEQ ID NO: 7 or biologically active variants of polypeptides having the amino acid sequence of SEQ ID NO: 6 or of SEQ ID NO: 7 can be used in the methods described herein.

Therefore, in a preferred embodiment, the polypeptide comprises or consist of an amino acid sequence which is at least 50%, 55%, 60%, 65%, 70%, 75%, 80%, 85%, 90%, 95%, 98% or more identical to the amino acid sequence of SEQ ID NO: 6 or to the amino acid sequence of SEQ ID NO: 7.

To determine the percent identity of two amino acid sequences or of two nucleic acid sequences, the sequences are aligned for optimal comparison purposes (e.g., gaps can be introduced in one or both of a first and a second amino acid or nucleic acid sequence for optimal alignment).

In a preferred embodiment, the polypeptide is encoded by a nucleic acid molecule comprising or consisting of a nucleotide sequence which is at least 50%, 55%, 60%, 65%, 70%, 75%, 80%, 85%, 90%, 95%, 98% or more identical to a nucleotide sequence comprising the nucleotide sequence of any one of SEQ ID NOs: 1, 2, 3, 4 or 5 or a complement thereof.

The residues at corresponding positions are then compared and when a position in one sequence is occupied by the same residue as the corresponding position in the other sequence, then the molecules are identical at that position. The percent identity between two sequences, therefore, is a function of the number of identical positions shared by two sequences (i.e., % identity = # of identical positions/total # of positions x 100). The percent identity between the two sequences is a function of the number of identical positions shared by the sequences, taking into account the number of gaps, and the length of each gap, which are introduced for optimal alignment of the two sequences.

The comparison of sequences and determination of percent identity between two sequences can be accomplished using a mathematical algorithm. A non-limiting example of a mathematical algorithm utilized for comparison of sequences is the algorithm of Karlin and Altschul (1990) Proc. Natl. Acad. Sci. USA 87:2264, modified as in Karlin and Altschul (1993) Proc. Natl. Acad. Sci. USA 90:5873. Such an algorithm is incorporated into the NBLAST and XBLAST programs (version 2.0) of Altschul, et al. (1990) J. Mol. Biol. 215:403. BLAST nucleotide searches can be performed with the NBLAST program score=100, wordlength=12 to obtain nucleotide sequences homologous to the nucleic acid molecules of the invention. BLAST protein searches can be performed with the XBLAST program, score=50, wordlength =3 to obtain amino acid sequences homologous to the protein molecules of the invention. To obtain gapped alignments for comparison purposes, Gapped BLAST can be utilized as described in Altschul et al., (1997) Nucleic Acids Research 25(17):3389. When utilizing BLAST and Gapped BLAST programs, the default parameters of the respective programs (e.g., XBLAST and NBLAST) can be used. See http://www.ncbi.nlm.nih.gov. Another preferred, non-limiting example of an algorithm utilized for the comparison of sequences is the algorithm of Myers and Miller, CABIOS (1989). Such an algorithm is incorporated into the ALIGN program (version 2.0 or 2.OU) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art, and include ADVANCE and ADAM, described in Torelli and Robotti (1994) Comput. Appl. Biosci. 10:3; and FASTA, described in Pearson and Lipman (1988) Proc. Natl. Acad. Sci. USA 85:2444.

In a further embodiment, the polypeptide has microcin-like, antimicrobial, antibacterial or antitumoral activity. The polypeptide is a bacterial polypeptide, preferably bacterial microcin S. Further, the polypeptide inhibits bacterial adherence to human intestinal epithelial cells. That is, it has to be understood that the polypeptide provides for antimicrobial/antibacterial activity against closely related species while the polypeptide does not exert antimicrobial/antibacterial activity against its producer who is resistant to the microcin it produces. Therefore, polypeptide inhibits the adherence of closely related bacterial species to human intestinal epithelial cells.

Preferably, the polypeptide is obtainable from an *E. coli* strain, more preferably from *E. coli* G3/10. In other embodiments, the *E. coli* G3/10 is *E. coli* G3/10 DSM 16443.

It is further preferred that the polypeptide is biodegradable.

### Nucleic acid molecules

The genome of the probiotic *E. coli* strain G3/10 was completely sequenced using the "*pyrosequencing*" technology and subsequently automatically annotated and further manually edited.

*E. coli* G3/10 has a 4935403 bp genome and six plasmids with a size of between 1.3 kb to 50 kb. 40 kb of the 50 kb plasmid called pSYM1 are almost completely identical (99% homology) to pMAS2027 from a uropathogenic *E. coli* (Ong, C. L., Beatson, S. A., McEwan, A. G. & Schembri, M. A. (2009) Appl Environ Microbiol 75, 6783-6791). The remaining 10 kb contain reading frames with no or only a low homology to nucleotide and amino acid sequences deposited in the databases of the NCBI and ExPASy. Four of these genes code for a completely new, not previously described *E. coli* microcin Class IIa including immunity and transport system. This microcin S gene cluster (SEQ ID NO: 1) consisting of the four clustered genes *mcsS* (SEQ ID NO: 2), *mcsI* (SEQ ID NO: 3), *mcsA* (SEQ ID NO: 4) and *mcsB* (SEQ ID NO: 5) has been isolated. This microcin was genotypically characterized and nominated microcin S. The two export genes of MccS are *mcsA* and *mcsB* (Fig. 2). Gene *mcsA* is a member of the *E. coli* hemolysin HlyD family and shows small homology to colicin secretion protein *cvaA.* Gene *mcsB* encodes an ABC-transporter with two regions of transmembrane domains and one peptidase domain.

A nucleic acid molecule of the present invention, e.g., a nucleic acid molecule having the nucleotide sequence of any one of SEQ ID NOs: 1 to 5 or of any one of SEQ ID NOs 8 to 26 or a portion thereof, can be isolated using standard molecular biology techniques and the sequence information provided herein. For example, using all or portion of the nucleic acid sequence of any one of SEQ ID NOs: 1 to 5 or of any one of SEQ ID NOs 8 to 26 as a hybridization probe, microcin S nucleic acid molecules can be isolated using standard hybridization and cloning techniques (e.g., as described in Sambrook, J. et al. Molecular Cloning: A Laboratory Manual. 2nd, ed., Cold Spring Harbor Laboratory, Cold Spring Harbor Laboratory Press, Cold Spring Harbor, NY, 1989).

A nucleic acid of the invention can be amplified using cDNA, mRNA or alternatively, genomic DNA, as a template and appropriate oligonucleotide primers according to standard PCR amplification techniques. Further useful primers are described herein below. The nucleic acid so amplified can be cloned into an appropriate vector and characterized by DNA sequence analysis. Furthermore, oligonucleotides corresponding to microcin S nucleotide sequences can be prepared by standard synthetic techniques, e.g., using an automated DNA synthesizer.

In a preferred embodiment, the nucleic acid molecule comprises or consists of a nucleotide sequence that is at least 50%, 55%, 60%, 65%, 70%, 75%, 80%, 85%, 90%, 95%, 98% or more identical to any one of SEQ ID NOs: 1, 2, 3, 4 or 5 or a complement thereof.

In another preferred embodiment, the nucleic acid molecule comprises a nucleotide sequence that encodes a polypeptide comprising or consisting of an amino acid sequence which is at least 50%, 55%, 60%, 65%, 70%, 75%, 80%, 85%, 90%, 95%, 98% or more identical to the amino acid sequence of SEQ ID NO: 6 or to the amino acid sequence of SEQ ID NO: 7.

In a further embodiment, the nucleic acid molecule encodes a bacterial polypeptide, preferably bacterial microcin S.

Preferably, the nucleic acid molecule is obtainable from an *E. coli* strain, more preferably from *E. coli* G3/10. In a specific embodiment, the nucleic acid molecule is obtainable from E. *coli* G3/10 DSM 16443.

### Nucleic acid primer or probe

The nucleotide sequence determined from the cloning of the microcin S genes allows for the generation of probes and primers designed for use in amplifying the microcin S gene of the invention and/or identifying microcin S genes in other species.

Hence, the invention further provides nucleic acid molecules as primers or probes which comprise a sequence that hybridizes to a nucleic acid molecule according to the invention under stringent conditions. Such a nucleic acid primer or probe comprises a sequence of at least 15 consecutive bases.

The nucleic acid primer or probe typically comprises a substantially purified oligonucleotide.

In a preferred embodiment, the nucleic acid primer or probe hybridizes to at least about 12 or 15, preferably about 20 or 25, more preferably about 30, 35, 40, 45, 50, 55, 60, 65, 75, or 100 consecutive nucleotides of a sense sequence of any one of SEQ ID NOs: 1, 2, 3, 4 or 5 or a complement thereof or of a naturally occurring allelic variant or mutant of any one of SEQ ID NOs: 1, 2, 3, 4 or 5. In one embodiment the nucleic acid primer or probe hybridizes to a nucleotide sequence of any one of SEQ ID NOs: 1, 2, 3, 4 or 5 or a complement thereof under stringent conditions.

In a more preferred embodiment the nucleic acid primer or probe comprises at least one of SEQ ID NO: 8 to SEQ ID NO: 26. The sequences of typical nucleic acid primers or probes are shown in Table 1.

**TABLE 1: Oligonucleotides (synthesized by Biomers, Germany)**

| **Oligonucleotide** | **Sequence (5'→ 3')** | **Function** |
|---|---|---|
| contig49_for SEQ ID NO: 8 | cagctggatatcctgcgcg | pSYM1 sequencing primer |
| contig49_rev SEQ ID NO: 9 | ggttgcccggcatccaacg | pSYM1 sequencing primer |
| pSYM1-SalIHF SEQ ID NO: 10 | tcaattgtgtcgactcaattactcttgtgag | pAZ6/pAZ8 cloning primer |
| pSYM1-NheI SEQ ID NO: 11 | catgtaatagtgctagcatgttaaaatttataag | pAZ6 cloning primer |
| pSYM1-NheIac SEQ ID NO: 12 | caaaaataatagctagcaagtgatgttttgtaatg | pAZB, pAZ12 cloning primer |
| ac-EcoRI SEQ ID NO: 13 | ctcgaattcatccattacaaaacatcac | pAZ9 cloning primer |
| ac-PstI SEQ ID NO: 14 | ctggctgcagtaattgttcaggaagtaacg | pAZ9 cloning primer |
| pSYM1_44-EcoRI SEQ ID NO: 15 | taggaattcagaggaactattggtggg | pAZ10 cloning primer |
| pSYM1_44-PstI SEQ ID NO: 16 | ctccgctgcagacttacttatcgactacaggtaccac | pAZ10 cloning primer |
| ab-EcoRI SEQ ID NO: 17 | gttagaattcataagagggatttttatgtcaaatatc | pAZ11 cloning primer |
| ab-PstI SEQ ID NO: 18 | gttgatactgcagcttatcgactacaggtaccacc | pAZ11 cloning primer |
| pAZ9-HindIII SEQ ID NO: 19 | cccaagcttagttaaatgtgctaatgctgtc | pAZ12 cloning primer |
| pAZ9-SalI SEQ ID NO: 20 | ggcatcggtcgacgcaac | pAZ12 cloning primer |
| pSYM1_43-NheI SEQ ID NO: 21 | cattgctagccatcacagataaactggataac | pAZ14 cloning primer |
| pSYM1_43-SalI SEQ ID NO: 22 | ccctgagtcgactcatggttataaaatattttg | pAZ13, pAZ14 cloning primer |
| recA-ff SEQ ID NO: 23 | atggctatcgacgaaaacaaac | Internal inhibition control |
| recA-rev SEQ ID NO: 24 | ttaaaaatcttcgttagtttctgc | Internal inhibition control |
| mcsS-ff SEQ ID NO: 25 | atgtcaaatatcagagaattgag | *mcsS* screening primer |
| mcsS-rev SEQ ID NO: 26 | ttatcgactacaggtaccacc | *mcsS* screening primer |

The nucleic acid primers or probes according to the invention are used to amplify or to screen for a nucleic acid molecule according to the invention.

Hence, the nucleic acid molecules or portions thereof may be amplified, e.g., by PCR or other amplification technique well known in the art, and the amplified nucleic acid molecules or portions thereof may be sequenced by methods known in the art. Polymerase chain reaction (PCR) is a method of amplifying nucleic acids, comprising denaturation, annealing and extension of nucleic acids, and during the process of annealing, hybridization of a probe and a target nucleic acid takes place. The conditions for the PCR can be altered according to the degree of homology between the probe and the target nucleic acid. When the annealing temperature is raised under given PCR conditions, the yield of non-specific hybridization products is decreased, whereas, when the annealing temperature is lowered, the yield of non-specific hybridization products is increased. See, e.g. J. Sambrook and David W. Russell: Molecular Cloning: A Laboratory Manual, Cold Spring Harbor Laboratory Press, (2000).

The probes disclosed herein can further be used to detect nucleic acid molecules of the invention in various cell types. For example, the nucleic acid primers or probes according to the invention may be used to screen for a nucleic acid molecule according to the invention using multiplex PCR. A multiplex PCR enables simultaneous amplification of different genes that are intended for amplification, in the same reaction. That is to say, different primer sets which are able to amplify their respective target sequences are placed in one reactor, and amplifications of the target sequences are simultaneously performed in a single PCR reaction (see e.g. Wittwer C.T., et al., Real-time multiplex PCR assays. Methods 25(4): 430-442, (2001); Markoulatos P., et al., Multiplex PCR: rapid DNA cycling in a conventional thermal cycler. J. Clin. Lab. Anal. 17(4): 108-112, (2003); O. Henegariu, et al., Multiplex PCR: Critical Parameters and Step-by-Step Protocol. BioTechniques 23: 504-511, (1997); and Markoulatos P., et al., Multiplex polymerase chain reaction: a practical approach. J. Clin. Lab. Anal. 16(1): 47-51, (2002).

### Plasmids

Plasmids are one type of a vector, which refers to a circular double stranded DNA loop into which additional DNA segments can be ligated. Certain plasmids are capable of autonomous replication in a host cell in which they occur or into which they are introduced (e.g., bacterial plasmids having a bacterial origin of replication and episomal mammalian vectors). Moreover, certain plasmids are capable of directing the expression of genes to which they are operatively linked. Such vectors are referred to herein as "*expression plasmids*".

*E. coli* G3/10 has six plasmids with a size of between 1.3 kb to 50 kb. 40 kb of the 50 kb plasmid called pSYM1 (Fig. 1) are almost completely identical (99% homology) to pMAS2027 from a uropathogenic *E. coli.* The remaining 10 kb contain reading frames with no or only a low homology to nucleotide and amino acid sequences deposited in the databases of the NCBI and ExPASy. Four of these genes code for a completely new, not previously described *E. coli* microcin Class IIa including immunity and transport system. This microcin S gene cluster (SEQ ID NO: 1) consisting of the four clustered genes *mcsS* (SEQ ID NO: 2), *mcsI* (SEQ ID NO: 3), *mcsA* (SEQ ID NO: 4) and *mcsB* (SEQ ID NO: 5) has been isolated.

Hence, a plasmid may comprise the nucleic acid molecule of the invention. In a preferred embodiment, the plasmid is an expression plasmid.

The expression plasmids of the invention comprise a nucleic acid of the invention in a form suitable for expression of the nucleic acid molecule in a cell, which means that the naturally occurring or recombinant expression vectors include one or more regulatory sequences, selected on the basis of the host cells to be used for expression, which is operatively linked to the nucleic acid sequence to be expressed.

Accordingly, it is further preferred that a regulatory element of the plasmid is operatively linked to the nucleic acid molecule of the invention.

In another embodiment, the plasmid is a conjugative plasmid.

Plasmids used herein are shown in Table 2:

**TABLE 2. Plasmids used**

| **Plasmid** | **Resistance** | **Relevant Genotype** | **Origin** |
|---|---|---|---|
| pSYM1 | | *mcsS, mcsI, mesA, mcsB* | this work |
| pST76-A | Amp^{r} | ori^{TS (30°C)} | Pósfai, G., M.D. Koob, H.A. Kirkpatrick, and F.R. Blattner. 1997. J. Bacteriol. 179: 4426-4428. |
| pSYM1-ST76An | Amp^{r} | *mcsS, mcsI, mcsA, mcsB* | this work |
| pAZ6 | Amp^{r} | *mcsS, mcsI, mcsA, mcsB* | this work |
| pAZ8 | Amp^{r} | *mcsI, mcsA, mcsB* | this work |
| pAZ9 | Cm^{r} | *mcsS* | this work |
| pAZ10 | Cm^{r} | ORF1 | this work |
| pAZ11 | Cm^{r} | ORF2 | this work |
| pAZ12 | Cm^{r} | *mcsS*₁₉₃₋₃₆₃ | this work |
| pAZ13 | Amp^{r} | *mcsI* | this work |
| pAZ14 | Amp^{r} | *mcsI*₃₆₁₋₆₅₁ | this work |

### Cells

A cell may be a host cell that can be can be any prokaryotic or eukaryotic cell. For example, a microcin S polypeptide can be expressed in bacterial cells such as *E. coli,* which is preferred herein, or insect cells, yeast or mammalian cells (such as Chinese hamster ovary cells (CHO) or COS cells). Other suitable host cells are known to those skilled in the art.

Plasmid DNA may be naturally occurring in the cell or may be introduced into prokaryotic or eukaryotic cells via conventional transformation or transfection techniques. Suitable methods for transforming or transfecting host cells can be found in Sambrook, et al. (Molecular Cloning: A Laboratory Manual. 2nd, ed., Cold Spring Harbor Laboratory, Cold Spring Harbor Laboratory Press, Cold Spring Harbor, NY, 1989), and other laboratory manuals.

Therefore, the present invention also relates to a cell comprising the nucleic acid molecule of the present invention, and/or the polypeptide of claim 1 and/ or a plasmid of the present invention.

The nucleic acid molecule may be integrated into the genome of the cell of the invention (subject cell) or, preferably into a plasmid in the subject cell.

It is preferred that the subject cell is a bacterial cell. For example, the cell is an *E. coli* cell, preferably from *E. coli* DSM 17252, most preferably an *E. coli* G3/10 cell.

The subject cell may also be a recombinant host cell.

In a preferred embodiment, the subject cell is a probiotic cell. Then, the cell has antimicrobial, antibacterial or antitumoral activity. It has to be understood that the cell provides for antimicrobial activity against closely related species while the cell does not exert antimicrobial activity against its producer who is resistant to the microcin it produces.

As such, the subject cell inhibits bacterial adherence to human intestinal epithelial cells.

### Antibodies

A microcin S immunogen typically is used to prepare antibodies by immunizing a suitable subject, (e.g., rabbit, goat, mouse or other mammal) with the immunogen. An appropriate immunogenic preparation can contain, for example, a naturally occurring or recombinantly expressed microcin S polypeptide or a chemically synthesized microcin S peptide. The preparation can further include an adjuvant, such as Freund's complete or incomplete adjuvant, or similar immunostimulatory agent. Immunization of a suitable subject with an immunogenic microcin S preparation induces a polyclonal anti-microcin S antibody response. Such methods are known in the art.

Hence, polyclonal anti-microcin S antibodies can be prepared as described above by immunizing a suitable subject with a microcin S immunogen. If desired, the antibody molecules directed against the microcin S polypeptide can be isolated from a mammal (e.g., from the blood) and further purified by well known techniques, such as protein A chromatography to obtain the IgG fraction. At an appropriate time after immunization, e.g., when the anti-microcin S antibody titers are highest, antibody-producing cells can be obtained from the subject and used to prepare monoclonal antibodies by standard techniques, such as the hybridoma technique originally described by Kohler and Milstein (1975) Nature 256:495-497 (see also, Brown et al. (1981) J. Immunol 127:539-46; Brown et al. (1980) J. Biol. Chem. 255:4980-83; Yeh et al. (1976) Proc. Natl. Acad. Sci. USA 76:2927-31; and Yeh et al. (1982) Int. J. Cancer 29:269-75), the more recent human B cell hybridoma technique (Kozbor et al. (1983) Immunol. Today 4:72), the EBV-hybridoma technique (Cole et al. (1985) Monoclonal Antibodies and Cancer Therapy, Alan R. Liss, Inc., pp. 77-96) or trioma techniques. The technology for producing monoclonal antibody hybridomas is well known (see generally Kenneth, R. H. in Monoclonal Antibodies: A New Dimension In Biological Analyses, Plenum Publishing Corp., New York, New York (1980); Lerner, E. A. (1981) Yale J. Biol. Med. 54:387-402; Gefter, M. L. et al. (1977) Somatic Cell Genet., 3:231-36). Briefly, an immortal cell line (typically a myeloma) is fused to lymphocytes (typically splenocytes) from a mammal immunized with a microcin S immunogen as described above, and the culture supernatants of the resulting hybridoma cells are screened to identify a hybridoma producing a monoclonal antibody that binds specifically to a microcin S polypeptide.

Accordingly, in embodiments, the present invention is also drawn to antibodies which selectively bind to a polypeptide that is a microcin S polypeptide. In view of the well established principle of immunologic cross-reactivity, the present invention therefore contemplates antigenically related variants of the polypeptide of SEQ ID NO: 6 or of SEQ ID NO: 7. An "*antigenically related variant*" is a polypeptide that includes at least a six amino acid residue sequence portion of a polypeptide of SEQ IS NO: 6 or of SEQ ID NO: 7 and which is capable of inducing antibody molecules that immunoreact with a polypeptide of SEQ ID NO: 6 or of SEQ ID NO: 7. Antibodies can be synthetic, monoclonal, or polyclonal and can be made by techniques well known in the art.

### Compositions and pharmaceutical compositions

The microcin S polypeptide or even a cell of the invention can be incorporated into a composition or a pharmaceutical composition which are suitable for administration. Accordingly, the present invention contemplates a composition comprising a polypeptide according to the invention or a cell according to the invention.

In a further preferred embodiment, the composition is an aqueous composition.

In a further embodiment, the composition has antimicrobial, antibacterial or antitumoral activity. Then, the composition inhibits bacterial adherence to human intestinal epithelial cells.

In a preferred embodiment, the composition is a probiotic composition. Preferably, the probiotic composition comprises a mixture of probiotic microorganisms, preferably of E. *coli* DSM 17252. For example, the composition comprises cells and/or autolysates of at least one of *E. coli* G1/2 (DSM 16441), G3/10 (DSM 16443), G4/9 (DSM 16444), G5 (DSM 16445), G6/7 (DSM 16446) and G8 (DSM 16448) or a mixture thereof. A composition may contain autolysates as well as cells in an amount of 3.00 x 10⁶ to 2.25 x 10⁸ cells per 1 ml, preferably 1.5 - 4.5 x 10⁷ cells per 1 ml. Preferably, the composition comprises autolysates as well as cells of *E. coli* bacteria and further additives. Accordingly, the invention provides a probiotic composition comprising at least one of the bacterial strains *E. coli* G1/2 (DSM 16441), G3/10 (DSM 16443), G4/9 (DSM 16444), G5 (DSM 16445), G6/7 (DSM 16446) and G8 (DSM 16448), i.e., one of the strains mentioned above or any bacterial strain selected by the method of the invention, where the composition comprises at least 1 strain, preferably from 2 to 3 strains, more preferably from 2 to 4 strains, even more preferred from 2 to 5 strains and most preferred from 2 to 6 strains, and where each of the strains is present in the composition in a proportion from 0.1 % to 99.9%, preferably from 1% to 99%, more preferably from 10% to 90%. In a preferred embodiment, the composition comprises at least one of the bacterial strains of the invention together with another strain or mixture of strains where the mixture comprises preferably from 2 to 6 strains, more preferably from 2 to 4 strains, most preferably from 2 to 3 strains and where each of the strains is present in the composition in a proportion from 0.1% to 99.9%, preferably from 1% to 99%, more preferably from 10% to 90%. The probiotic compositions of the invention are preferably in a lyophilized form, in a frost form or even dead. In a preferred embodiment, a probiotic composition comprises one or more probiotic microorganisms and a carrier which functions to transport the one or more probiotic microorganisms to the gastrointestinal tract, the carrier may comprise modified or unmodified resistant starch in the form of high amylose starches or mixtures thereof. The carrier acts as a growth or maintenance medium for microorganisms in the gastrointestinal tract such that the probiotic microorganisms are protected during passage to the large bowel or other regions of the gastrointestinal tract.

In a preferred embodiment, the composition may further be a pharmaceutical composition. Then, the pharmaceutical composition further comprises a pharmaceutically acceptable carrier. The pharmaceutical composition may contain a therapeutically effective amount of the polypeptide or the cell and one or more adjuvants, excipients, carriers, and/or diluents. Acceptable diluents, carriers and excipients typically do not adversely affect a recipient's homeostasis (e.g., electrolyte balance). Acceptable carriers include biocompatible, inert or bioabsorbable salts, buffering agents, oligo- or polysaccharides, polymers, viscosity-improving agents, preservatives and the like. Further details on techniques for formulation and administration of pharmaceutical compositions can be found in, e.g., REMINGTON'S PHARMACEUTICAL SCIENCES (Maack Publishing Co., Easton, Pa.).

Example of additives include glucose, lactose, sucrose, mannitol, starch, cellulose or cellulose derivatives, magnesium stearate, stearic acid, sodium saccharin, talcum, magnesium carbonate and the like. Examples of additives that may be added to provide desirable color, taste, stability, buffering capacity, dispersion or other known desirable features are red iron oxide, silica gel, sodium lauryl sulfate, titanium dioxide, edible white ink and the like. Similar diluents can be used to make compressed tablets.

In embodiments, supplementary active compounds can also be incorporated into the compositions.

Pharmaceutical compositions of the invention are formulated to be compatible with its intended route of administration. Examples of routes of administration include parenteral, e.g., intravenous, intradermal, subcutaneous, oral, transdermal (topical), transmucosal, and rectal administration.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Oral administration may be applied in the form of a capsule, liquid, tablet, pill, or prolonged release formulation.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier, wherein the compound in the fluid carrier is applied orally and swished and expectorated or swallowed. Pharmaceutically compatible binding agents, and/or adjuvant materials can be included as part of the composition. The oral composition can contain any of the following ingredients, or compounds of a similar nature: a salt such as sodium chloride or magnesium sulfate, such as magnesium sulfate · 7 H₂O, potassium chloride, calcium chloride, such as calcium chloride · 2 H₂O, magnesium chloride, such as magnesium chloride · 6 H₂O, purified water, a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

In an alternative embodiment, the composition may be a disinfectant for surfaces. Hence, the use of the composition for desinfection of surfaces also contemplated herein.

In a preferred embodiment, the disinfection composition further comprises whenever appropriate, a surface-active substance (tenside) or substance mixture, aromatic substances, adjuvants and binders.

In another embodiment, the disinfection composition further comprises a binder and optionally aromatic and coloring substances and adjuvants such as substances for softening the water, fillers and the like, and is available in liquid form, as tablets or in granulate form.

Preferably, the disinfection composition is food-safe.

The disinfection composition further protects against pathogenic microbials with an antibiotic resistance.

### Kit

Kits comprising, in one or more containers, a therapeutically or prophylactically effective amount of the composition of the invention are also provided. The kit may optionally include instructions for performing the intended medical uses as disclosed herein or for performing the methods as provided.

### Medical uses of the polypeptide, the cell, the compositions and the kit

Since the polypeptide; the cell and the composition display antimicrobial, antibacterial or antitumoral activity, the invention further relates to their medical uses in therapy or prevention.

Specifically, they may be used in treating or preventing microbial infections.

In a preferred embodiment, the microbial infection comprises infections with enteropathogenic and/or enterohemorrhagic *E. coli* (EPEC, EHEC). In a further embodiment, the microbial infection comprises hemolytic-uremic syndrome (HUS), preferably enteropathic hemolytic-uremic syndrome.

In a preferred embodiment, the microbial infection comprises an enterohemorrhagic *E. coli* (EHEC) infection. That is, the enterohemorrhagic *E. coli* O104:H4 outbreak strain or any other EHEC susceptible to microcin S. In a further preferred embodiment, the microbial infection comprises a Shiga toxin producing *E. coli* infection. The Shiga toxin producing *E. coli* may be an enterohemorrhagic *E. coli.* Beside other virulence factors Shiga toxins (Stx 1 ; Stx 2; Stx 1,2) are responsible for severe, mostly bloody diarrhea and onset of the haemolytic uremic syndrome. EHEC cells release higher amounts of Shiga toxins by treatment with certain antibiotics. Therefore, antibiotic-treatment of patients is discussed to be contraindicated. By treating the microbial infection using the subject polypeptide; the subject cell or the composition of the invention the production of Shiga toxin can be inhibited.

In other embodiments, the microbial infection comprises an enteropathogenic *E. coli* infection.

In embodiments the microbial infection comprises enhanced-spectrum beta-lactamase - producing enterobacteriaceae infection. Preferably, the microbial infection comprises an enhanced-spectrum beta-lactamase-producing *E. coli.*

In embodiments, the subject polypeptide; the subject cell or the composition of the invention inhibit the microbials causing the microbial infection in a proportion of 1:1000, preferably 1:500, more preferably 1:100, even more preferably 1:50, most preferred 1:20 or even 1:1 to 1:10.

In an alternative embodiment, the subject polypeptide; the subject cell; the composition of the invention or the kit of the invention are for use in treating functional gastrointestinal disorders, preferably irritable bowel syndrome. It is preferred that they are used in treating adults and children.

In another alternative embodiment, the polypeptide; the cell; the composition or the kit are for use in treating a tumor. They are capable, even if to varying degrees, of inhibiting the growth of tumor cells. Preferably, a microcin S polypeptide may induce apoptosis in human cell lines. For example, the polypeptide, the nucleic acid molecule, the cell or a composition according to the invention may be applied to a subject using administration routes as described herein, e.g. intravenously. The polypeptide, the nucleic acid molecule or the cell may accumulate in a tumor. If the subject cell is administered, the cell may accumulate in the tumor and produce the polypeptide according to the invention. The polypeptide may also be expressed from the subject nucleic acid molecule or may be released from the composition. Microcin S may be placed under the control of a tumor active promoter.The polypeptide; the cell or the composition are administered to subjects in a biologically compatible form suitable for pharmaceutical administration.

The polypeptide; the cell; the composition or the kit should induce at significant levels the apoptosis in tumoral cells, while not giving rise to any effect on the normal cell systems.

Non-limiting examples of diseases or disorders which can be treated and/or prevented by using the polypeptide; the cell; the composition or the kit are tumors in general in particular colon-rectal cancer, cancer of the liver, gliomae, neuroblastomae, squamocellular oral carcinoma, lymphoid tumors, cancer of the prostate gland, cancer of the bladder, cancer of the breast, cancer of the pleura and the peritoneum.

In some embodiments described herein, the polypeptide; the cell or the composition, can be administered to a subject in parenteral, e.g., intravenous, intradermal, subcutaneous, oral, transdermal (topical), transmucosal, and rectal, preferably in an oral form.

The polypeptide; the cell or the composition, may be administered to a subject in a pharmaceutically effective amount. Administration of a pharmaceutically effective amount of the polypeptide; the cell or the compositions of the present invention is defined as an amount effective, at dosages and for periods of time necessary to achieve the desired result. For example, a pharmaceutically effective amount of a polypeptide; a cell or a composition may vary according to factors such as the disease state, age, sex, and weight of the individual, and the ability of the polypeptide; the cell or the compositions to elicit a desired response in the individual. Dosage regime may be adjusted to provide the optimum therapeutic response. For example, several divided doses may be administered daily or the dose may be proportionally reduced as indicated by the exigencies of the therapeutic situation.

In order to optimize therapeutic efficacy, a microcin S polypeptide is administered at different dosing regimens at different points of time.

The subject can be administered a single pharmaceutically effective dose or multiple pharmaceutically effective doses, e.g., 2, 3, 4, 5, 6, 7, or more. Specifically, the subject may be administered a single pharmaceutically effective dose 2, 3, 4, 5, 6, 7, or more times a day.

Specifically, the subject can be administered a dose of 5-15 droplets of an aqueous composition. More preferably a dose of 10 droplets are to be administered. In such an embodiment, 1 ml contains about 14 droplets.

A pharmaceutically effective amount (i. e., a pharmaceutically effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight.

Administrations of multiple doses can be separated by intervals of hours, days, weeks, or months. In further embodiments, they are administered at least one time, two times or three times a day with meals within water, preferably three times a day with meals within water.

They can optionally be administered to the subject for a limited period of time and/or in a limited number of doses. For example, in some embodiments administration to the subject can be terminated (i.e., no further administrations provided) within, e.g., one year, six months, one month, or two weeks. For example, the provided administration may be terminated after six months. In chronic diseases, it may be necessary to increase the period to up to six months.

In some embodiments, the dose may be increased after 2 days, 3 days, 4 days, 5 days, 6 days, 1 week, 2 weeks, three weeks, four weeks or more. The dose to be administered to a subject may be increased to 15-25 droplets, more preferably to 20 droplets, of an aqueous composition.

In children, they may be administered in an oral form of 5-15 droplets of an aqueous composition. More preferably 10 droplets are to be administered to a child. In further embodiments, they are administered to a child at least one time, two times or three times a day with meals within water, preferably one time a day with meals within water.

In all medical use embodiments, the polypeptide; the cell; the composition or the kit of the invention, are to be administered at the beginning of the treatment to an adult 10 droplets three times a day with meals within water and the dose is increased after 1 week to 20 droplets and to a child 10 droplets once per day with meals within water.

### Process for making the microcin S polypeptide

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture).

Specifically, a cell of the invention, such as a prokaryotic or eukaryotic host cell in culture, can be used to produce (i.e., express) a microcin S polypeptide. Accordingly, the invention further provides methods for producing a microcin S polypeptide using the cells of the invention.

A method for producing the subject polypeptide, microcin S, is contemplated herein. Such a method comprises: using a nucleic acid molecule, a nucleic acid primer or probe, a plasmid or a cell according to the invention, or

synthesizing the polypeptide according to the invention by means of solution phase or solid phase peptide synthesis techniques.

In a preferred embodiment, the method comprises the steps of
i) providing a cell comprising the nucleic acid molecule of the invention or the plasmid of the invention,
ii) culturing the cell under conditions that permit expression of the polypeptide from the nucleic acid molecule.

In a preferred embodiment, the method further comprises the step of (iii) recovering the polypeptide produced.

The isolated subject polypeptide is thus produced by the said method.

In some embodiments, microcin S polypeptides are produced by naturally occurring cells or, alternatively, by recombinant DNA techniques. For example, a nucleic acid molecule encoding a microcin S polypeptide may already be encoded by a plasmid or can be inserted into a plasmid, e.g., an expression plasmid. Using the plasmid, the nucleic acid molecule can be introduced into a cell by recombinant methods. When expressed in a cell, the cell is preferably cultured under conditions allowing for expression of a microcin S polypeptide. The microcin S polypeptide can be recovered from a cell suspension if desired. As used herein, "*recovered*" means that the microcin S polypeptide is removed from those components of a cell or culture medium in which it is present prior to the recovery process. The recovery process may include one or more refolding or purification steps. Buffers and methods for inducing folding of a denatured microcin S polypeptide are known in the art.

The polypeptides within the scope of the present invention may be synthesized using standard solution-phase or solid-phase techniques known in the art (e.g. R. B. Merrifield (1963). "Solid Phase Peptide Synthesis. I. The Synthesis of a Tetrapeptide". J. Am. Chem. Soc. 85 (14): 2149-2154). Thus, the subject polypeptide can be produced-by-condensing a partial peptide or amino acid. When the partial peptide or amino acid has a protective group, the protective group is detached whereupon the subject polypeptide is produced. Solid-phase, that is a resin, synthesis is commenced from amino acids or peptides whose α-amino groups and functional groups of side-chain have been suitably protected. They are condensed on the resin according to the sequence of the subject polypeptide by various condensation techniques which are known per se. At the end of the series of reactions, the peptide or the protected peptide is removed from the resin and the protective groups are removed to obtain the objective polypeptide. For example, solid-phase synthesis is commenced from the carboxy-terminal end of the peptide using a protected amino acid. BOC or FMOC protective groups can be used for all amino groups even through other protective groups are suitable. For example, BOC-lys-OH can be esterified to chloromethylated polystyrene resin supports. The polystyrene resin support is preferably a copolymer of styrene with about 0.5 to 2% divinylbenzene as a crosslinking agent which causes the polystyrene polymer to be completely insoluble in certain organic solvents. See Stewart et al., Solid-Phase Peptide Synthesis (1969), W.H. Freeman Co., San Francisco; and Merrifield, J. Am. Chem. Soc. (1963) 85:2149-2154. These and other methods of peptide synthesis are also exemplified by U.S. Patent Nos. 3,862,925; 3,842,067; 3,972,859; and 4,105,602.

The polypeptide synthesis may use manual techniques or automatically employing, for example, an Applied Biosystems 403A Peptide Synthesizer (Foster City, California) or a Biosearch SAM II automatic peptide synthesizer (Biosearch, Inc., San Rafael, California), following the instructions provided in the instruction manual supplied by the manufacturer.

### Process for preserving food

The invention also relates to a method for preserving food, the method comprising:
adding to food at least one kind of a natural antimicrobial agent, wherein the agent is
   a) the polypeptide according the invention;
   b) the cell according to the invention; and/or
   c) the composition according to the invention.

Since Gram positive and Gram negative bacteria are almost always found together in foods, the polypeptides, the nucleic acid molecules, the cell or the compositions within the scope of the present invention show effectiveness towards bacteria, e.g. *Salmonella, Escherichia coli, Klebsiella, Pseudomonas or Listeria.* More specifically bacteria such as *Salmonella typhimurium,* other species of *Escherichia coli, Klebsiella pneumoniae, Pseudomonas aeruginosa, Bacteroides gingivalis, Listeria monocytogenes* and others.

It is to be understood that the agent is added in an amount sufficient to inhibit the contaminant bacteria.

Preferably, the polypeptide, the nucleic acid molecule, the cell or the composition added to a food achieves a protective effect. In one embodiment, the polypeptide, the cell or the composition do not affect the consumption of food. For example, they are tasteless and nontoxic to humans and animals.

Hence, the polypeptides, the nucleic acid molecules, the cell or the compositions within the scope of the present invention are particularly suited for the control and prevention of contamination of raw ingredients, processed foods and beverages by bacterial pathogens and other microbial spoilage organisms. They may be used in connection with a food product that is susceptible to bacterial growth or degradation. Potential food related uses include treatment of meats, especially poultry, eggs, cheese, dairy products, fruits, vegetable derived products, grains and grain derived products, canned foods, salad dressing, fermented beverages and seafood, such as fish, or many other foods. Examples of dairy foods include, but are not limited to, cheese, milk, cream, and fermented dairy foods such as yogurt. Examples of meats include, for instance, ham, beef, salami, chicken, and turkey, including whole parts or processed meat products made therefrom. Other food products include processed food products including ready to eat meals, entrees, and meats, deli salads, mayonnaise, dressings (including salad dressings), sauces and condiments, pastas, soups, edible oils, fish and fish products, egg products, beverages, aseptically packaged foods, as well as mixtures of the foregoing. Other food related uses include treatment of food packaging and handling equipment. Further uses include as food preservative, such as in processed cheese, cream, milk, dairy products and in cleaning poultry, fish, meats or vegetables.

The polypeptides, the nucleic acid molecules, the cell or the compositions of the present invention may be used by mixing with and/or applying on a blendable food product, but may be applied to a surface of solid food products by a dip, rinse, or spray, or by application to the interior of such products, e.g. by injection. They may be applied as a marinade, breading, seasoning rub, glaze, colorant mixture, and the like, or as an ingredient to be mixed with and incorporated into the food product. In still other aspects, the polypeptides, the nucleic acid molecules, the cell or the compositions may be indirectly placed into contact with the food surface by applying the composition to food packaging materials, such as a casing or a film, and thereafter applying the packaging to the food surface such that the composition comes into contact with the external food surface. The optimum amount to be used will depend upon the particular food product to be treated and the method used to apply the composition to the food and/or the food surface, but can be determined by routine experimentation.

Advantages are that the polypeptides, the cell or the compositions of the present invention are easy to digest, nontoxic, may not induce allergies, and are difficult for pathogenic microbials to develop resistance against.

### Process for identifying bacteria that are sensitive to the microcin S polypeptide:

The invention further relates to an *in vitro* method for identifying bacteria which are sensitive to the microcin S polypeptide. In such a method the subject polypeptide; the subject nucleic acid molecule, the subject nucleic acid primer or probe, the subject cell, the subject antibody or the subject composition may be used.

In one embodiment, the method comprises the step of bringing pathogenic bacteria causing a microbial infection in contact with the subject polypeptide; the subject nucleic acid molecule, the subject nucleic acid primer or probe, the subject cell, the subject antibody or the subject composition.

It has to be understood that microcin S can be added to the pathogenic bacteria in the form of a subject polypeptide. However, also a subject nucleic acid molecule may be added from which microcin S may be expressed. It is further possible to add a subject cell which produces microcin S. Finally, the subject composition comprising microcin S may be added to the pathogenic bacteria.

In a preferred embodiment, the method comprises the steps of
providing a first set of cells;
pre-incubating a first set of cells with the subject polypeptide; the subject nucleic acid molecule, the subject cell, the subject antibody or the subject composition;
providing a second set of cells;
infection of the first set of cells and the second set of cells with pathogenic bacteria causing a microbial infection;
measuring adherence efficiency of the first set of cells and the second set of cells;
comparing the adherence efficiency of the first set of cells with the adherence efficiency of the second set of cells,
wherein, when the adherence efficiency of the first set of cells is lower than that of the second set of cells, then the cells of the first set of cells are sensitive to microcin S.

A reduction adherence efficiency of a first set of cells compared to a corresponding system of second set of cells, wherein microcin S has not been added in the form of a subject polypeptide; a subject nucleic acid molecule, a subject cell or the subject composition being indicative for the sensitivity of the first set of cells to microcin S.

In preferred embodiments the cells of the first set of cells and of the second set of cells are human intestinal epithelial cells.

In a preferred embodiment, the *in vitro* method for identifying bacteria is a modified agar diffusion test. In a first step, the bacteria causing a microbial infection are applied to a culture plate. In a second step, a filter-paper disk is impregnated with the subject polypeptide; the subject nucleic acid molecule, the subject nucleic acid primer or probe, the subject cell, the subject antibody or the subject composition. Then, the filter paper is placed on the surface of the agar on the culture plate.

This has the effect, that microcin S diffuses from the filter paper into the agar. A concentration gradient is established, wherein the concentration of microcin S is highest next to the disk, and decreases with distance to the disk.

If microcin S is effective against the bacteria at a certain concentration, no colonies will grow where the concentration in the agar is greater than or equal to the effective concentration. This is the zone of inhibition. Thus, the size of the zone of inhibition is a measure of the compound's effectiveness: the larger the clear area around the filter disk, the more effective the compound.

### Method for coating dressing material:

Within the scope of the invention is a method for coating dressing material, wherein dressing material is coated with
a) the polypeptide according to the invention; and/or
b) the composition according to the invention.

Since the new microcin S polypeptide of the present invention shows antimicrobial effects it may be used for coating dressing material. It should be understood that coated dressings for groups of subjects, e.g. such as for patients with first and second-degree burns, is advantageous. Such subjects need protection from bacterial infections. In a preferred embodiment, the dressing material is antiseptic dressings. In a more preferred embodiment antiseptic dressings are patches or bandages. In embodiments, the coated dressing material provides for protection against *Pseudomonas aeruginosa,* which is a pathogen associated with burn wounds.

### Examples

### Example 1: Bacterial strains and growth conditions

Bacterial strains used herein are listed in Table 3.

**TABLE 3: Bacterial strains used**

| **Strain** | **Relevant Genotype** | **Origin** |
|---|---|---|
| *E. coli* Nissle 1917 | wild-type | Mutaflor^{®} |
| | | |
| *E. coli* G1/2^{a} | wild-type | Symbioflor 2^{®}, |
| | | SymbioPharm |
| | | |
| *E. coli* G3/10^{a} | wild-type | Symbioflor 2^{®}, |
| | | SymbioPharm |
| | | |
| *E. coli* G4/9^{a} | wild-type | Symbioflor 2^{®}, |
| | | SymbioPharm |
| | | |
| *E. coli* G5^{a} | wild-type | Symbioflor 2^{®}, |
| | | SymbioPharm |
| | | |
| *E. coli* G6/7^{a} | wild-type | Symbioflor 2^{®}, |
| | | SymbioPharm |
| | | |
| *E. coli* G8^{a} | wild-type | Symbioflor 2^{®}, |
| | | SymbioPharm |
| | | |
| *E. coli* MDS42 | K-12 multiple deletion strain | Posfai, G., G. Plunkett III; T. Feher, D. Frisch, G.M. Keil; K. Umenhoffer, V. Kolisnychenko, B. Stahl, S.S. Sharma, M. de Arruda, V. Burland, S.W. Harcum, and F.R. Blattner. 2006. Emergent Properties of Reduced-Genome Escherichia coli. Science 312: 1044-1046. |
| | | |
| EPEC E2348/69 O127:H6 | Amp^{r} (pUC19), Kana^{r} | Donnenberg, M. S. and Kaper, J. B. 1992. Enteropathogenic *Escherichia coli.* Infect. Immun. 60: 3953-3961. |
| | (pUC4k) or Cm^{r} | |
| | (pACYC184) | |
| | | |
| | | |
| | | |
| EHEC 86-24 O157:H7 | *stx2, eaeA, EHEC-hlyA, astA,* | Griffin, P.M., S.M. |
| | Sm^{r} | Ostroff, R.V. Tauxe, K.D. |
| | | Greene, J.G. Wells, J.H. |
| | | Lewis, and P.A. Blake. |
| | | 1988. Illnesses associated |
| | | with *Escherichia coli* |
| | | 0157:H7 infections. A |
| | | broad clinical spectrum. |
| | | Ann. Intern. Med. |
| | | 109:705-712. |
| | | |
| EHEC O104:H4 | wild-type, *stx2,* ESBL (*ctxM*) | human isolate, Dresden |
| | | |
| EHEC O104:H4 | wild-type, *stx2,* ESBL (*ctxM*) | human isolate, Frankfurt |
| | | (Oder) |
| | | |
| EHEC O128:H2 | *stx1, stx2, EHEC-hlyA,* Amp^{r} | human isolate, Frankfurt |
| | (pUC4k) | (Oder) |

| | | |
|---|---|---|
| ^{a} DSM17252 | | |

### Example 2: Identification of a microcin-encoding gene cluster in E. coli G3/10

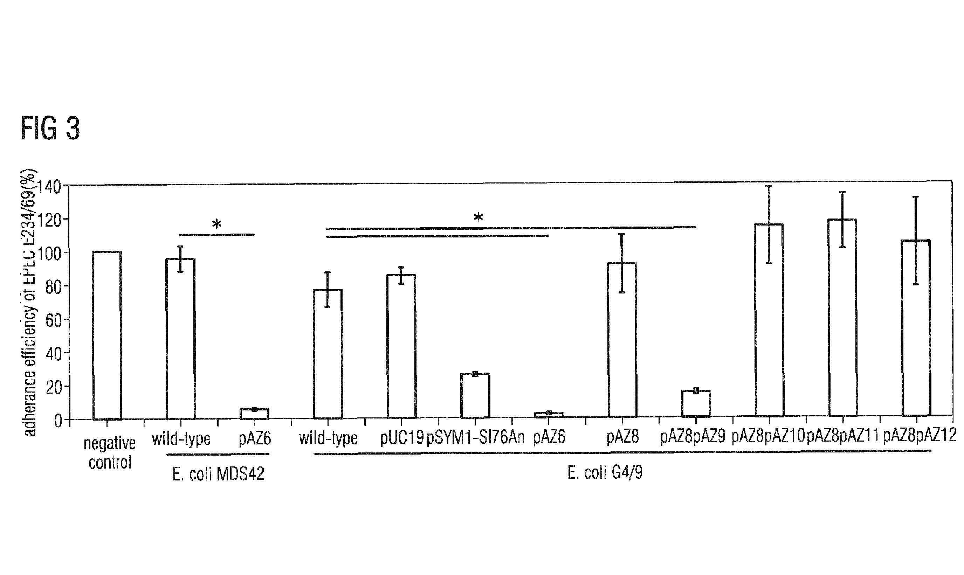

The genomes of six *E. coli: E. coli* G1/2, G3/10, G4/9, G5, G6/7 and G8. were sequenced. Annotation revealed no known microcin within the genome of *E. coli* G3/10. *E. coli* G3/10 contains a large conjugative plasmid pSYM1 (Fig. 1) with a size of 50.6 kb. The plasmid is 99% identical to plasmid pMAS2027 of an uropathogenic *E. coli* isolate. Moreover, it contains a 10 kb insertion fragment, but BLAST analysis revealed only uncharacterized and unnamed genes. To identify the origin of bactericidal action it was tried to cure the strain *E. coli* G3/10 from its megaplasmid pSYM1. Despite performing many of common curing procedures, for example mitomycin C or heat treatment, the strain could not be cured. Therefore, plasmid pSYM1 was transferred to *E. coli* G4/9 by conjugation. To allow a screening of conjugants, at first an ampicillin resistance cassette was integrated into pSYM1 resulting in pSYM1-ST76An. The resulting *E. coli* G4/9 pSYM1-ST76An was able to inhibit EPEC adherence significantly (Fig. 3). It was concluded that plasmid pSYM1 carries genes that are responsible for the shown effect. A 4.7 bp fragment of plasmid pSYM1 was cloned into pBR322 resulting in plasmid pAZ6, which was then transformed into *E. coli* G4/9. The pAZ6 enables *E. coli* G4/9 to inhibit EPEC adherence efficiency significantly (Fig. 3) indicating that the 4.7 kb fragment of pSYM1 is responsible for the EPEC adherence inhibition effect of *E. coli* G3/10 (Fig. 2). BLAST analysis of automatically annotated open reading frames (ORF) revealed small homologies to characterized proteins or protein families indicating a relationship to microcins-encoding operons. Nevertheless, the microcin itself remained undetected. Three genes, named *mcsI, mcsA* and *mcsB,* were cloned into pBR322 resulting in pAZ8. Small ORFs upstream of this operon (Fig. 2), which are possible microcin-encoding genes, were cloned into pACYC184 and subcloned into *E. coli* G4/9 pAZ8. With this step a plasmid-separated encoding of the probable microcin and microcin-helper proteins was facilitated. Only *E. coli* G4/9 containing plasmids pAZ8 and pAZ9 significantly inhibited EPEC adherence, whereas G4/9 pAZ8 affects EPEC adherence similar to G4/9 wild-type (Fig. 3). The small gene, nominated *mcsS,* encodes a microcin, which was named microcin S.

### Example 3: Functional characterization of microcin S and elucidating its self immunity

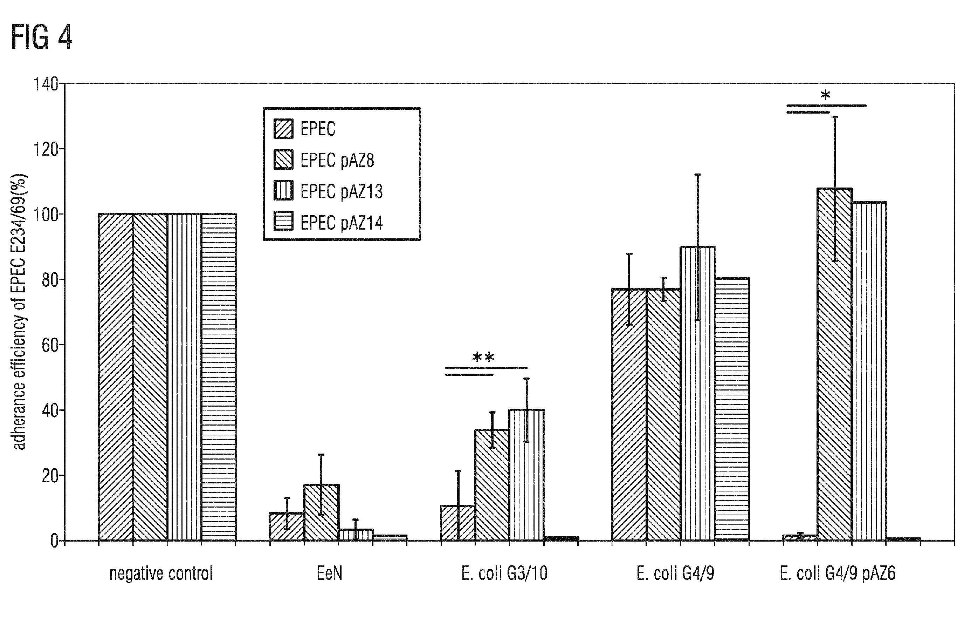

Bacterial adhesion is a crucial first step of many infectious diseases. Therefore, a test system quantifying the inhibition of adherence to human intestinal epithelial cells is suitable to demonstrate a beneficial effect to the host. In a first experiment, confluent monolayers of CACO-2 or LOVO cells were pre-incubated with bacterial test strains EcN, *E. coli* G3/10, E. *coli* G4/9 or *E. coli* G4/9 pAZ6 at an MOI of 100:1 *E. coli* to host cells. After two hours of incubation, cells were washed and infected with EPEC E2348/69 using an MOI of 100:1 EPEC to host cells. *E. coli* G3/10 and *E. coli* G4/9 pAZ6 were capable of inhibiting EPEC adherence similar to EcN. EcN adherence inhibition was shown to be dependent on the strains microcins activity. Adherence efficiency in % are expressed as adherence of EPEC relative to adherence without any pre-incubation (negative control), which is set 100 %. The data are the mean ± SD of at least three separate experiments in duplicate wells. Fig. 4 displays the adherence efficiency of EPEC E2348/69 wild-type and plasmid-complemented mutants carrying *mcsI* to human intestinal epithelial cells after pre-incubation with different microcin expressing (*E. coli* G3/10, G4/9 pAZ6 and EcN) and non-expressing (*E. coli* G4/9) strains. As a result, *mcsI* could be undoubtedly identified as the microcin S self-immunity gene.

### Example 4: Impact on enterohemorrhagic E. coli

Enterohemorrhagic *E. coli* adhere to intestinal epithelial cells. In a second experiment, confluent monolayers of LOVO cells were pre-incubated with bacterial test strains at a multiplicity of infection (MOI) of 100:1 *E. coli* to host cells. The influence of EcN and *E. coli* G3/10 was tested. After two hours of incubation, cells were washed and infected with EHEC using an MOI of 100:1 EHEC to host cells. Three EHEC isolates were tested as indicator strains using the *in vitro* adherence assay. We used HUS-associated EHEC 86-24 serotype O157:H7 and two different isolates of the EHEC O104:H4 outbreak strain as well as an EHEC O128:H2 patient isolate. Since the EHEC O104:H4 isolates are ESBL producers, medical treatment is limited. Adherence efficiency in % are expressed as adherence of EHEC relative to adherence without any pre-incubation (negative control), which is set 100 %. The data are the mean ± SD of three separate experiments in duplicate wells. EHEC adherence was only inhibited by *E. coli* G3/10, which indicates a different mechanism of action of MccM/H47 from EcN and MccS from *E. coli* G3/10. Fig. 5 displays the adherence efficiency of EHEC strains 86-24 serotype O157:H7 (A), EHEC O104:H4 isolated in Dresden (B) and EHECs O104:H4 (C) and O128:H2 (D) isolated in Frankfurt (Oder) into human intestinal epithelial cells after pre-incubation with EcN or *E. coli* G3/10.

### Example 5: Screening for the microcin S gene mccS in various E. coli and other enterobacteriaceae

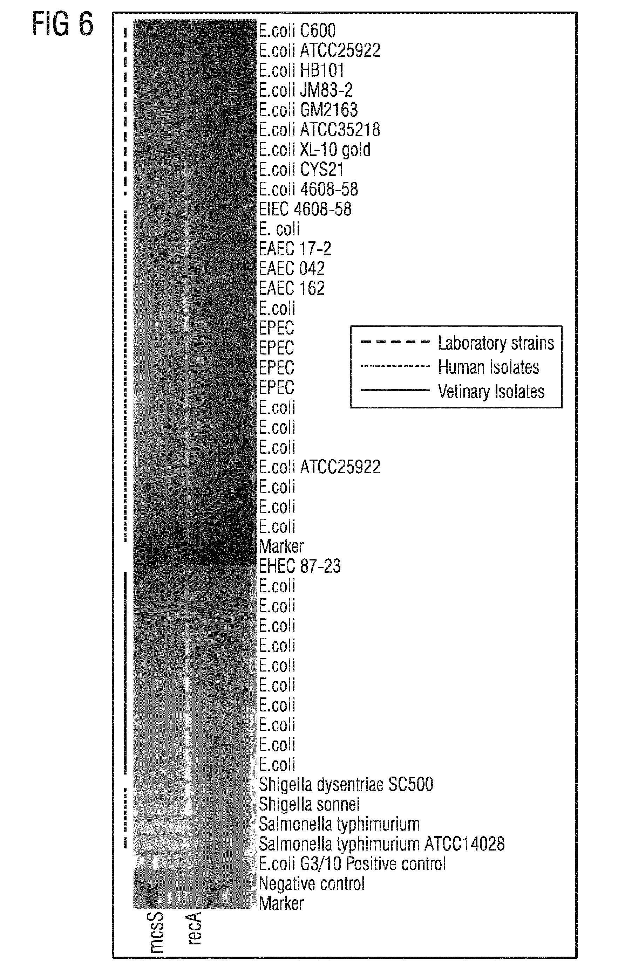

The sequence of microcin S is unknown in nucleotide and amino acid databases. Screening for the MccS gene cluster was performed in 38 *E. coli,* 2 *Shigella* and 2 *Salmonella* strains using multiplex PCR and the gene *recA* as inhibition control. The tested strains are common laboratory strains, human clinical isolates and veterinary isolates of different origin. The McsS gene could not be detected in any of the tested 42 human and veterinary isolates as well as laboratory strains. Results are depicted in Fig. 6.

## Claims

1. An isolated polypeptide, wherein the polypeptide:
a) comprises an amino acid sequence which is at least 50% or more identical to the amino acid sequence of SEQ ID NO: 6 or of SEQ ID NO: 7;
b) is encoded by a nucleic acid molecule comprising a nucleotide sequence which is at least 50% or more identical to a nucleotide sequence comprising the nucleotide sequence of any one of SEQ ID NOs: 1, 2, 3, 4 or 5 or a complement thereof;
c) is encoded by a nucleic acid molecule which hybridizes to a nucleotide sequence complementary to a nucleotide sequence comprising the sequence of nucleotides of any one of SEQ ID NOs: 1, 2, 3, 4 or 5 or a complement thereof under stringent conditions; or
d) comprises a naturally occurring allelic variant of a polypeptide comprising the amino acid sequence of SEQ ID NO: 6 or of SEQ ID NO: 7.

2. An isolated nucleic acid molecule encoding a microcin S polypeptide, wherein said nucleic acid molecule:
a) comprises a nucleotide sequence that is at least 50% or more identical to any one of SEQ ID NOs: 1, 2, 3, 4 or 5 or a complement thereof,
b) comprises a nucleic acid molecule which hybridizes to a nucleotide sequence complementary to a nucleotide sequence comprising the nucleotide sequence of any one of SEQ ID NOs: 1, 2, 3, 4 or 5 or a complement thereof under stringent conditions;
c) comprises a nucleotide sequence that encodes a polypeptide comprising an amino acid sequence which is at least 50% or more identical to the amino acid sequence of SEQ ID NO: 6 or of SEQ ID NO: 7; or
d) comprises a nucleic acid molecule which encodes a naturally occurring allelic variant of a polypeptide comprising the amino acid sequence of SEQ ID NO: 6 or of SEQ ID NO: 7.

3. A nucleic acid primer or probe comprising a sequence hybridizing to the nucleic acid molecule according to claim 2 under stringent conditions.

4. A plasmid comprising the nucleic acid molecule according to claim 2.

5. A cell comprising the nucleic acid molecule according to claim 2 and/or the polypeptide of claim 1 and/or the plasmid of claim 4.

6. An antibody which selectively binds to a polypeptide according to claim 1.

7. A composition comprising
a) the polypeptide according to claim 1; and/or
b) the cell according to claim 5.

8. The composition according to claim 7, wherein the composition is a pharmaceutical composition further comprising a pharmaceutically acceptable carrier.

9. The composition according to claim 7, wherein the composition is a disinfectant for surfaces.

10. The polypeptide according to claim 1; the cell according to claims 5; the composition according to claim 7 for use in therapy or prevention.

11. The polypeptide according to claim 1, the cell according to claim 5 or the composition according to claim 7 for use in treating or preventing microbial infections, preferably the microbial infection comprises infections with enteropathogenic and/or enterohemorrhagic *E. coli* (EPEC, EHEC) or preferably the microbial infection comprises hemolytic-uremic syndrome (HUS).

12. The polypeptide according to claim 1; the cell according to claim 5 or the composition according to claim 7 for use in treating or preventing gastrointestinal disorders, preferably functional gastrointestinal disorders.

13. The polypeptide according to claim 1; the cell according to claim 5 or the composition according to claim 7 for use in treating a tumor.

14. A method for producing the polypeptide according to claim 1, the method comprising:
using the nucleic acid molecule according to claim 2, the nucleic acid primer or probe according to claim 3, the plasmid according to claim 4 or the cell according to claim 5 or
synthesizing the polypeptide according to claim 1 by means of solution phase or solid phase peptide synthesis techniques.

15. An isolated polypeptide produced by the method according to the method of claim 14.

16. An *in vitro* method for identifying bacteria which are sensitive to microcin S, the method comprising: using the polypeptide according to claim 1; the nucleic acid molecule according to claim 2, the nucleic acid primer or probe according to claim 3, the cell according to claim 5, the antibody according to claim 6 or the composition according to claim 7.

17. A method for preserving food, the method comprising:
adding to food at least one kind of a natural antimicrobial agent, wherein the agent is
a) the polypeptide according to claim 1;
b) the cell according to claim 5; and/or
c) the composition according to claim 7.

18. A method for coating dressing material, wherein
dressing material is coated with:
a) the polypeptide according to claim 1; and/or
b) the composition according to claim 7.
